# FASCICULE DE BREVET EUROPEEN

(11) **EP 0 828 857 B1**
(45) Date de publication et mention de la délivrance du brevet: **12.06.2002**
(21) Numéro de dépôt: 97916487.8
(22) Date de dépôt: 24.03.1997
(51) Int. Cl.: C12Q 1/68

(54) **SONDE, SEQUENCE NUCLEIQUES ET PROCEDE NOTAMMENT, DE DETECTION ET D'IDENTIFICATION DE LEVURES, DE MEME QUE LES OUTILS GENETIQUES CORRESPONDANTS**
SONDE, NUKLEINSÄURESEQUENZEN, BESONDERS GEEIGNETE METHODE ZUR DETEKTION UND IDENTIFIZIERUNG VON HEFEN, SOWIE ENTSPRECHENDE GENETISCHE HILFSMITTEL
PROBE, NUCLEIC ACID SEQUENCES, METHOD PARTICULARLY SUITABLE FOR DETECTING AND IDENTIFYING YEASTS, AND GENETIC TOOLS THEREFOR

(30) Priorité: 22.03.1996 FR 9603835
(43) Date de publication de la demande: 18.03.1998
(73) Titulaire: BIO MERIEUX, 69280 Marcy l'Etoile (FR)
(72) Inventeur: CARLOTTI, Arnaud, F-69003 Lyon (FR); VILLARD, Jean, F-69008 Lyon (FR)
(74) Mandataire: Fleurance, Raphael
(86) Numéro de dépôt international: FR9700518
(87) Numéro de publication internationale: WO9736003

(56) Documents cités:
- WO-A-93/23568
- WO-A-95/11991
- APPLIED AND ENVIRONMENTAL MICROBIOLOGY, vol. 62, no. 1, Janvier 1996, WASHINGTON US, pages 41-46, XP000196695 M.M. BALEIRAS COUTO ET AL.: "Evaluation of molecular typing techniques to assign genetic diversity among Saccharomyces cerevisiae strains" cité dans la demande
- JOURNAL OF CLINICAL MICROBIOLOGY, vol. 34, no. 7, Juillet 1996, WASHINGTON US, pages 1726-1731, XP000196696 A. CARLOTTI ET AL.: "Species-specific identification of Candida krusei by hybridization with the CkF1,2 DNA probe"

### - LISTE DE SEQUENCES

(1) INFORMATIONS GENERALES:
   (i) DEPOSANT:
      (A) NOM: Université Claude Bernard Lyon I
      (B) RUE: 43, Boulevard du 11 Novembre 1918
      (C) VILLE: Villeurbanne
      (E) PAYS: France
      (F) CODE POSTAL: 69622 Cedex
   (ii) TITRE DE L' INVENTION: Sonde et procédé notamment pour la détection et/ou l'identification inter/infraspécifique de levures, de même que les outils génétiques correspondants
   (iii) NOMBRE DE SEQUENCES: 5
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0. Version #1.30 (OEB)
   (vi) DONNEES DE LA DEMANDE ANTERIEURE:
      (A) NUMERO DE LA DEMANDE: FR 96 03835
      (B) DATE DE DEPOT: 22-MAR-1996
(2) INFORMATIONS POUR LA SEQ ID NO: 1:
   (i) CARACTERISTIQUES DE LA SEQUENCE:
      (A) LONGUEUR: 5261 paires de bases
      (B) TYPE: nuclotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 1:
   GAATTCGGTA AGCGTTGGAT TGTTCACCCA CTAATAGGGA ACGTGAGCTG GGTTTAGACC     60
   GTCGTGAGAC AGGTTAGTTT TACCCTACTG ATGAATGTTG TCGCAATAGT AATTGAACTT    120
   AGTACGAGAG GAACCGTTCA TTCAGATAAT TGGTTTTTGC GGCTGTCTGA GCAGACACTG    180
   CCGCGACGCT ACCATCTGCT GGATAATGGC TGAACGCCTC TAAGTCAGAA TCCATGCTAG 240
   AACGCGACGA TTACCTGCCC TCGCACATTT GAGAAGGATA CGAATAAGGC CCTGTGGCCG 300
   CAGAACCGTA GCAGGCCGGC AGCGGTGCGC ATGGCGGAAA GGCCGTGTGT GCTTGCCGGC 360
   GGATGGCAAT GTCAGGATGC GCTCAGATAA ATCCTATGCA TACGACTTAG ATGTACAACG 420
   GGGTATTGTA AGCAGTAGAG TAGCCTTGTT GTTACGATCT GCTGAGATTA AGCCTCTGTT 480
   GTCCGATTTG TTGCTTCGGC GGCGAAGGAG CGGTCCGTAG AGACGGGCTG TTGTTTCGGG 540
   AGTGTGGGGA GTGTGGGGGG ACTTAGGAGC GGGGGTAGTG TGGGGGAGTG TGGGGACTTG 600
   GGAGCGGGNG TAGTAGGTGG TACTGATGGT ACGGGGAGGA GCCTACCCTT TTTTGTCGTT 660
   TTGGCAAAGA CATTGCAGGA TTTACAAAGT GATTTACGAT ATGAACTAGG ACTATGTTGT 720
   GTGGAGGGGG TGGGGCTGTA GAGAGGATGG ATTTGACGGG CATGACGGGG ACGGCTTGGA 780
   GGCCGCCCAT GTCGTATGCC CCGGGGTAGG GTATAGGCCG GGAGGGGCCA GTTGGGAAAA 840
   TAGCGAAGAG GGGGGGAGGA GGCTAAGAAG GCGGTGGGGG AGGGGGTAGG TTGATAAGTA 900
   GGGGGTGTAG GAGCGTGGGT GGAGTGGGAG TGGGAGTGGG TGGTACAGAG TGGGTGGTAC 960
   AGAGTGGGTG GTACAGAGTA CAGAGTGGGG AGAGTACAGA GTGGGAGTGG TAGGAGGAAG 1020
   AGACAGTACA CAGTAGGAGG AAGAGAGTGG TAGGAAGAGA GTGGTAGGAG GAAGAGCGGC 1080
   AGGAGGAAGA GAGTGGTAGG AGGAAGAGTG GTAGGAGGAA GAGTGGTAGG AAGAGAGTGG 1140
   TAGGAGGAAG ATGGAATCCT AGACCTTGTA AATTCAGACA AAACTCGGTC AAAAACTAGA 1200
   CAATGGGGGC AGTGCGGGAT TTTCGACAAC AAAAACAAAG GGAGGTAAAC AAAGGCAAAC 1260
   AAAGGTAAAC ACAAGAAAAA CAGGCAGCAC AGGCAGNAAA AAGATTGCAG CACCTGAGTT 1320
   TCGCATATGG TCTCCCACTA CACTACTCGG TCAGGCTCTT AGCAGCTTAA CTACAGTTGA 1380
   TCGGACGGTA AACGGTGCTT TCTGCTAGAT ATGGCCGCAA CCGAAAGTAT AGAACGGGCG 1440
   GCGTATGGGA GGTTTGATTT TGGGCGGGCA GATTTCGACG GGTCGATAAA TCTCGACGGG 1500
   ACGCATGTTT GCAGGGCCAT AGCAGACGAA CCAGAGCAGC TGAACAGGCG AGTCAACCAC 1560
   CGTTGGAAAG AGCGCCGAGA AGTATCTAGT CCCCCCCATC GGTGTGGCCA TAGGACGCAA 1620
   TCGGCAACAT GGCCAAAATC AAAAAACAAA AATTCCCACT TTTACAAAAT ATATATAATT 1680
   TACAAAAAAT AGGTCTGGCA AAAATGCAAT TTTGGAAAAA AATCGAGTAA AAAGTAAATA 1740
   TTATGGGCGG AACGGATAGC CGTCGACGCG ACGATGGTGA AAACCGTGGT TGGAAAGCCG 1800
   ATATCGGGCC TGGGCGGAGA AAACGGCGAA AAGACAACTC CGAACACGAC ATTTCGGCAA 1860
   TGTTGCTGTT TCGGCAAAGT CCGAGGCGAA GTCCGAGGCG TAACTCCGAT GGGCGGAGAT 1920
   AAAGTGCGCA CGTGACTGGT GCGAGGGGTG GGGCGTTCTG GAGCGACGCA AAAAAAAAAA 1980
   CACGCACAAA AAAAATGGAC GTCGAGAAAA AAAAATTTAC ACGAGCGAGC ACTAAGGTGG 2040
   GAATGGACGT CCCCAGAAAT GTCTTCCACT TTGCCACCAC TTTTTCTTTT GCACACTTCT 2100
   GCTGTATGTC TGTCTGCACC ACCTCTTCCA CAACCAAGCA CACCACAGGT AGGATACTAA 2160
   CCACAGCAGG GCTGTCTCTG CAGCCAACCA CCCAGCAGTT TCTTTTGATT TTGATTTTTT 2220
   TGATTTTNNT GATTTTTATT TTGACTGATT TTGCTTTTCC CTTGTGTAAA AGTCTCGTCG 2280
   ACATTGCAGC TGGATGCAAT GGGCGTTTCT GAACACCAAT CCACTTAGTT GGGTTGATTT 2340
   TTCTTACTGT CTGATGAAGA TATGATTGTT TTTTAAAATT CCATTTTTTT TGATTTTATA 2400
   TAACTTCACT GTGGGGTGGT TTCCCTTGGC CTGCAAGCTC ATACACATTA CAGTTCCATC 2460
   CATTGCACGT TTCTAAACAC TACTCCACTT AGTTGGGCTG TTTATTTCCA CATGCTGATG 2520
   AAATGGTTGC TGTTTTTCAG AATTTCAGTT TTTTGATTTT ATATAACTTC ACTTTGGAGA 2580
   TGATTTCCCT TGGACTTTAC AATCACACAC ATTACAGTTC CATCCATTGC ACGTTTCTAA 2640
   ACACTACTCC ACTTAGTTGG GCTATTTATT TCCACATGCT GATGAAATGG TTGCTGTTTT 2700
   TCAGAATTTC AGTTTTTTTG ATTTTATATA ACTTCACTTT GGAGATGATT TCCCTTGGAC 2760
   TTTACAATCA CACACATTAC AGTTCCATCC ATTGCACGTT TCTAAACACT ACTCCACTTA 2820
   GTTGGGCTAT TTATTTCCAC ATGCTGATGA AATGGTTGCT GTTTTTCAGA ATTTCAGTTT 2880
   TTTTGATTTT ATATAACTTC ACTTTGGAGA TGATTTCCCT TGGACTTTAC AATCACACAC 2940
   AITACAGTTC CATCCATTGC ACGTTTCTAA ACACTACTCC ACTTAGTTGG GCTATTTATT 3000
   TCCACATGCT GATGAAATGG TTGCTGTTTT TCAGAATTTC AGTTTTTTTG ATTTTATATA 3060
   ACTTCACTTT GGAGATGATT TCCCTTGGAC TTTACAATCA CACACATTAC AGTTCCATCC 3120
   ATTGCACGTT TCTAAACACT ACTCCACTTA GTTGGGCTGT TTATTTCCAC AGGTTGATGA 3180
   AATGGTTGCT GTTTTTCAGA ATTTCAGTTT TTACAATTTC AACTAGTTTC ACTTTGGAGA 3240
   TGATTTCCCT TGGCATGGAA GCTCACACAC ATTACAGTTC CATCCATTGC ACGTTTCTAA 3300
   ACACTACTCC ACTTAGTTGG GCTGTTTATT TCCACAGGTT GATGAAATGG TTGCTGTTTT 3360
   TCAGAATTTC AGTTTTTTTG ATTTTATATA ACTTCACTTT GGAGATGATT TCCCTTGGAC 3420
   TTTACAATCA CACACATTAC AGTTCCATCA ATTACACGTT TCTAAACACT ACTCCACTTA 3480
   GTTGGGCTAT TTATTTCCAC AGGTTGATGA AGTTGTCAAG TTTTACAATT TTGGTTGCTG 3540
   AAGGTTCGGC AATTCACTGT TGTATAATTG AACGACAGAC ACTTCAATCA GCACTGTGCT 3600
   TCCCTTTTAT GTATTCTCCA ACGTAGTATC TTGACAAAGT ATATATATGA CTGCCACTTT 3660
   GATTATTTTG ATAGCTTTGT ATTTGACCAC TAATTTTGTA TTTGACCACC AATTTGAAAA 3720
   TGCCAATACC AATTTGCATT TGCCAGGGAA TTTCAATACC AATTTGCATT GCCAGGGAAT 3780
   TTCAATACCA ATTTCCAGGA ATTTCAATAC TAATTACCAC CATTTCCAGG AATTAATTTC 3840
   GTCGAAAAAT TAAAAAAGNC GTCGAGAAAT TTTGTGTTTG TTTTGCAAAC AAAATATCAC 3900
   ACACAACACT GAATACACGG AAGTTCTGTT GGACACAGAG CCCCACACAC ACAACGTACA 3960
   TGCAATTGGA AGGTATGTAT GTGTTGGCCA GGAGCAGGCG CAACTGTGCC GTGCTGCTTG 4020
   TGCATAATAC AGCGGCGTAC CGACAGAAAT AGAGCCCCCC AAAATTTGAT GGAAAAATAG 4080
   TGGACAGTTG GACTTTGCAA GTTGGACGCA GTGGAATAGA CAACCAAACA CCCCGATTAA 4140
   CAAGCCGATG GCCGATTGAC CCAATGCCAA TCAGTATGGA TCGATAACTG TCCAACTGAA 4200
   CGAGTAACAA GAAGATGTTT TCCAAATGTC AGAATTTCAA ATCGGATGTT CCACTGATGG 4260
   TCTGGGCGTT GCAGTCTGGG CTCCAGGTGT GTCAACTGGA CATTGCCAAG CGTGCACCGA 4320
   TGTTGCAAGA CGATTACCAC GGCAACAAAC CGGTGTTTAC GACAAGAACA CTGGCAGATG 4380
   CATCGAATAC TGATTTGGTG CAATTTCAAG GTTTGGGAGT TTTGTATAAT GTGTGTATAT 4440
   TATGATGCGA CAACATGCGC CGGTGTGTAA ATAGTGTATT GCAACCTGTA AATAAACTAA 4500
   ATCGACAAGG AATTGGTGGA ATTAGACGTA AAATAGTGTT GGCGAAAATA CGTTTTTTGT 4560
   TTCTGGGGGA CCTATGACGA TACTTTTTGG ACGCACATGT GGAGCGGCAT GAAATTAGTG 4620
   TGTGGCCGAG TTTGGGAGGA TATTCCCCAG GCGGTTGCCA AGATATTTGA AATAATCTGA 4680
   ATTTCAAAAA TGTCGGTGCT CTTTCGAAAT GCCATGTCCA GAGGATTTGA AATTTTTGGA 4740
   TGGAGCTGTG TTGGCTGGGT TGATGTTAAG GGAATGATAT ATCTGCGCTT TTCAATGTAT 4800
   TATGTTTCAG ATTTTTTGAG ATTTTTGAAT CTTTGAATTT TTTGAAATCT TTTGAATTTT 4860
   TTGAATCTTT TGAATTTTTT GAAATCTTTT GAATTTTTTT CAAAGTCTAT AAATGTTATT 4920
   GTGTTTTTGG TTTTATTTTA GCTTTTGCAA AATGTTATTT CATTATACAG TTCAAAATAA 4980
   TTTAATTGCC TATATGTTTT ACAATATAAT ATATTTTATT TCAGATTTCA AAATAATTTT 5040
   TTCCATTAAA CGTTTATAAT ACATTATTTT ACTTCAGATT TCAGGATATA CTATTCATTC 5100
   CCACACTTTA AAATATATTA TTCAATTTCA CACACCAAAG TGTACTATTT AACTTCACAT 5160
   TTCAAAATAA AATATTTGAC AATACCTAAA TATGGCATTT TTATTTCGGA TTTCGTTAGA 5220
   TATTCTTTAG TTATACAATC CCAAACATGC TATTCGAATT C 5261
(2) INFORMATIONS POUR LA SEQ ID NO: 2:
   (i) CARACTERISTIQUES DE LA SEQUENCE:
      (A) LONGUEUR; 1257 paires de bases
      (B) TYPE: nucléotide
      (C) NOMBRE DE BRINS: simple
      (D) CONFIGURATION:- linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 2:
(2) INFORMATIONS POUR LA SEQ ID NO: 3:
   (i) CARACTERISTIQUES DE LA SEQUENCE:
      (A) LONGUEUR: 81 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 3:
   TTTTGAATCT TTGAATTTTT TGAAATCTTT TGAATTTTTT GAATCTTTTG AATTTTTTGA 60
   AATCTTTTGA ATTTTTTTCA A 81
(2) INFORMATIONS POUR LA SEQ ID NO: 4:
   (i) CARACTERISTIQUES DE LA SEQUENCE:
      (A) LONGUEUR: 89 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 4:
   AGTGGGAGTG GGAGTGGGTG GTACAGAGTG GGTGGTACAG AGTGGGTGGT ACAGAGTACA 60
   GAGTGGGGAG AGTACAGAGT GGGAGTGGT 89
(2) INFORMATIONS POUR LA SEQ ID NO: 5:
   (i) CARACTERISTIQUES DE LA SEQUENCE:
      (A) LONGUEUR: 140 paires de bases
      (B) TYPE: nucléotide
      (C) NOMBRE DE BRINS: simple
      (D) CONFIGURATION: linéaire
   (ii) TYPE DE MOLECULE: ADN (génamique)
   (iii) HYPOTHETIQUE: NON
   (iv) ANTI-SENS: NON
   (xi) DESCRIPTION DE LA SEQUENCE: SEQ ID NO: 5:
   AGGAGGAAGA GACAGTACAC AGTAGGAGGA AGAGAGTGGT AGGAAGAGAG TGGTAGGAGG 60
   AAGAGCGGCA GGAGGAAGAG AGTGGTAGGA GGAAGAGTGG TAGGAGGAAG AGTGGTAGGA 120
   AGAGAGTGGT AGGAGGAAGA 140

## Description

### DOMAINE TECHNIQUE :

La présente invention concerne la détection et la caractérisation de levures. En particulier elle fournit des sondes spécifiques, notamment nucléiques, et un modèle de développement des dites sondes pour la détection et, en particulier, l'identification et le typage (caractérisation infra-spécifique) des souches de levures de différentes espèces d'intérêt médical et industriel.

### ART ANTERIEUR :

Les levures sont des champignons, micro-organsimes eucaryotes, chez lesquels la forme unicellulaire est prédominante (Barnett J., W., R., W. Payne and D. Yarrow., "Yeasts charactéristics and identification, 2nd Edition, Cambridge university press, Cambridge, 1991). Elles sont divisées en genres (e.g. *Candida*) et espèces (e.g. *Candida krusei*) sur la base de critères de reproduction sexuée (lorsqu'elle existe), morphologiques, physiologiques, chimiotaxonomiques et moléculaires.

Les levures sont ubiquitaires. Généralement leur innocuité est bien établie. Elles sont souvent utilisées comme agent de transformation (auxiliaires technologiques) dans les procédés agro-alimentaires, mais certaines sont parfois pathogènes. Les levures sont généralement considérées comme étant des pathogènes opportunistes.

Les levures pathogènes opportunistes intéressent le domaine de la mycologie médicale. Ces levures sont responsables d'infection superficielles, cutanées, sous-cutanées, et systémiques. Ces dernières sont les plus sérieuses en raison des taux de mortalité élevés qui leur sont imputés. Les levures pathogènes appartiennent le plus souvent au genre *Candida*. Parmi ce genre les espèces plus particulièrement importantes sont *Candida albicans*, *Candida krusei*, *Candida tropicalis, Candida lusitaniae, Candida (Torulopsis) glabrata*. Les infections qu'elles provoquent sont appelées "Candidoses".

La prévalence et la pathogénécité de ces levures opportunistes sont élevées chez les sujets immunodéprimés, notamment chez les patients neutropéniques, atteints de SIDA, cancéreux, etc...

Toute la difficulté du traitement de Ce type d'infections, provient du fait que certaines espèces sont résistantes à certains antifongiques, notamment au fluconazole (e.g. *C. krusei, C. glabrata*). En outre, pour une même espèce certaines souches présentent un caractère de résistance alors que d'autres sont sensibles aux antifongiques considérés (e.g. *C. albicans, C. lusitaniae*).

En conséquence, une détection rapide et une identification précise et fiable ainsi qu'un typage des souches de ces espèces sont de plus en plus nécessaires, notamment pour permettre la mise en place d'un traitement antifongique approprié. Il existe, en outre, un réel besoin en marqueurs épidémiologiques fiables, à des fins d'études épidémiologiques dans le domaine médical, ou de traçage des souches dans les industries agroalimentaires ou de fermentation.

Mais force est de constater que, les méthodes classiques d'identification des levures ne donnent pas entière satisfaction à tous ces égards.

On connaît ainsi notamment celles décrites par Barnett J., W., R. W. Payne and D. Yarrow., dans "Yeasts characteristics and identification", 2nd Edition, Cambridge university press, Cambridge 1991, qui nécessitent un isolement préalable du micro-organisme en culture pure, puis l'étude de ses caractéristiques morphologiques et surtout physiologiques (plus de 80 tests), l'ensemble de ces opérations requérant de 10 à 30 jours pour une identification. Ces méthodes sont donc fastidieuses et particulièrement longues. Elles sont réservées aux laboratoires de référence et inutilisables en pratique courante.

Plus rapide et plus rationnelles, les méthodes miniaturisées et standardisées d'identification des levures d'importance médicale, comprennent cependant toujours un isolement préalable puis une identification du micro-organisme à l'aide de tests physiologiques, réalisés par exemple à l'aide de galeries. La durée de tels tests est d'environ 5 jours pour aboutir à une identification. De plus ces techniques donnent parfois des résultats équivoques. Des espèces telles que *C. krusei, C. inconspicua, C. lipolytica, C. norvegensis, C. rugosa* et *C. valida* sont parfois, voire même souvent, confondues.

Avec le progrès de la biologie moléculaire, une nouvelle technologie utilisant le principe d'hybridation ADN-ADN ou ADN-ARN s'est développée pour la mise au point des tests d'identification rapides, sensibles et spécifiques. Le matériel génétique des micro-organismes présents dans l'échantillon est decelé directement à l'aide de sondes nucléiques à ADN ou ARN marqué. La détection et l'identification peuvent être réalisées simultanément sans isolement préalable.

Les rares méthodes décrites d'identification des levures à l'aide de sondes nucléiques, présentent l'inconvénient d'être basées sur des sondes de petites tailles (30 à 35 nucléotides) qui nécessitent la mise en oeuvre d'un marquage radioactif plus sensible que le marquage non radioactif mais aussi plus contraignant.

Un autre inconvénient de ces sondes lié à leur taille réduite, s'exprime dans le cadre de leur mise en oeuvre dans les techniques de polymérisation en chaine (PCR). Ces dernières consistent à amplifier une séquence ADN donnée, en la multipliant à partir d'un couple d'amorces et l'aide d'une polymérase. De telles amplifications peuvent être employées dans un but diagnostique, afin de faciliter la détection. Dès lors que ces sondes seraient utilisées comme amorces en PCR cela limiterait les possibilités de choix des amorces dans une petite séquence de 30 à 35 nucléotides.

Ces sondes sont généralement dirigées contre des séquences cibles d'ADN codant la petite sous unité 18 S de l'ARN ribosomique (ssuARNr). Ces cibles sont classiquement choisies car les séquences d'ADN codant les ARN ribosomaux sont les premières à avoir été déterminées. De même l'utilisation de la partie 3' des séquences codant la grande sous unité 25 S de l'ARN ribosomique (1suARNr) a été décrite. Il existe un problème lié au choix de séquences codant les ssuARNr ou 1suARNr comme cibles. En effet ces séquences ont un caractère universel, c'est-à-dire qu'une partie importante d'entre elles est retrouvée chez tous les micro-organismes (levures, bactéries) ; elles sont dites "hyper-conservées". Le choix des séquences cibles est donc limitées aux portions limitées variables de ces séquences codantes des ARNr.

En outre, parmi les sondes décrites dans l'art antérieur, aucune ne permet le typage des souches, c'est-à-dire la différenciation des individus au sein de l'espèce, ces individus étant uniquement assignés à un groupe : e.g. l'espèce *Candida kruse*i, l'espèce *Candida glabrata*, l'espèce *Candida lusitaniae*, etc... En effet, les sondes préalablement décrites ne permettent pas un typage des souches au sein des espèces car elles ne révèlent pas suffisamment de polymorphisme de la taille des fragments de restriction parmi les souches de ces espèces et ainsi ne constituent pas de marqueurs épidèmiologiques fiables.

A titre d'illustrations de telles sondes connues et répondant imparfaitement aux besoins techniques existant en la matière, on peut citer:
les sondes oligonucléotidiques développées par la société Genetrak Systems et dirigées contre des séquences de 30 (probes 1351), 33 (probe 1537) et 35 (probe 1530) nucléotides de l'ADN codant l'ARN ribosomal 18 S. Ces sondes sont décrites dans la demande de brevet européen N° 0 422 869. Elles ont été testées contre 4 souches de *Candida krusei*. Il est à noter qu'il n'est pas fait état dans les exemples de cette demande, de détection de *Candida krusei* par hybridation dans les prélèvements de sang humain, crachats ou liquide céphalorachidien. Par ailleurs, ces sondes ne permettent pas de différencier les souches entre elles au sein de l'espèce *Candida krusei*.

- la sonde décrite par Niesters et collaborateurs (Niesters et al., 1993, Rapid polymerase chain reaction-based identification assays for Candida species. J. Clin. Microbiol., 31, 904-910) 1993). Il s'agit d'une sonde de 20 nucléotides (oligonucléotide 705) dirigée contre une séquence spécifique du produit d'amplification de la petite sous unité d'ARN ribosomal (ssuARNr). Cette étude est utilisée dans une méthode d'identification des espèces du genre *Candida* basée sur l'amplification en chaîne (PCR) de séquences du gène codant la ssuARNr, puis le séquençage direct de ces séquences. Cette méthodologie concerne, là encore, l'ARN ribosomal. Elle présente l'inconvénient de nécessiter un séquençage direct du produit d'amplification, technique qui n'est accessible qu'à un nombre limité de laboratoires de recherche et surement pas applicable en routine. De plus, d'après les auteurs, l'oligonucléotide 705 ne permet pas une amplification spécifique de l'ADN de *Candida krusei*. Les sondes décrites par Niesters et collaborateurs ne permettent pas elles non plus de typer les souches de *Candida krusei*.

On connaît également, notamment au travers de l'article rédigé par BALEIRAS COUTO et al - Applied & Environmental Microbiology, Jan. 1996 p.41-46 "Evaluation of molecular typing techniques to assign genetic diversity among *Saccharomyces cerevisiae* strains". Cet article divulgue la discrimination de souches à l'intérieur de l'espèce *Saccharomyces cerevisiae*, par recours aux techniques d'identification du type de celles faisant intervenir des fragments d'ADN polymorphiques amplifiés de manière aléatoire (RAPD) et du type réaction de polymérisation en chaîne (PCR) à l'aide d'amorces nucléotidiques. Les fragments obtenus par ces techniques sont ensuite soumis à des digestions à l'aide d'enzyme de restriction. Toutes les séquences et fragments ainsi obtenus sont testés dans l'identification infraspécifique de 16 souches différentes de *Saccharomyces cerevisiae*. La RAPD et la PCR ont permis de reconstituer les régions d'espacements intergéniques transcrites (IGR - ITS) et les régions intergéniques non transcrites (IGR - NTS) de cistrons d'ADNr codant pour la synthèse d'ARN ribosomique (ARNr). Ce document ne décrit nullement le contenu des séquences reconstituées par RAPD et PCR dont la longueur est d'ailleurs d'au moins 7 kbases (kb). Cet article enseigne qu'il n'existe pas de polymorphisme significatif entre les souches de *Saccharomyces cerevisiae* A. Dans le meilleur des cas (correspondant à celui où l'on utilise des *Taq*I), il n'est possible de discriminer que 6 souches sur 16. Ce résultat est tout-à-fait perfectible.
Par ailleurs, BALEIRAS COUTO et al incitent plutôt le lecteur à s'intéresser aux régions intergéniques d'espacement transcrite (ITS), pour la différenciation infraspécifique de levure. Il présente ces régions intergéniques transcrites comme étant plus appropriées que les régions intergéniques non transcrites (NTS).
En outre, les amorces (Primers) utilisées dans la technique de (PCR) préconisées dans ce document, ne fonctionnent pas de façon universelle sur toutes les espèces de levures, ceci constitue un facteur limitatif de la technique. En particulier les auteurs indiquent que l'amplification n'a pas été possible pour les espèces *C. valida, C. krusei, Z. bailii*, ou *Z. rouxii*.
Il apparaît donc que les techniques décrites dans cet article ne conviennent que pour du typage infraspécifique de levure et notamment pas pour l'identification d'espèces de levure.

La demande de brevet internationale WO 95/11 991 décrit une sonde et un procédé de détection des levures de l'espèce *C. krusei*. Les outils génétiques constituant cette sonde sont formés par un fragment d'ADN et/ou d'ARN de taille comprise entre 7 et 4 kb, hybridant spécifiquement avec l'ADN et/ou l'ARN de *Candida kruse*i et ne codant pas pour l'ARN ribosomal (ARNr).
Sont également compris dans ces outils génétiques, les produits de transcription et de traduction du susdit fragment, de même que les associations entre ces différents outils. Cette demande de brevet ne divulgue pas la structure des fragments d'ADN mis en oeuvre dans cette sonde. Le moyen de caractérisation utilisé pour ces fragments, est formé par leur carte de restriction enzymatique. L'espèce de levure exclusivement concernée par ce document est *Candida krusei*.

La demande PCT WO-A-93 23 568 (A.R. HOLMES et al) concerne des méthodes de diagnostic des infections fongiques, selon lesquelles on met en oeuvre des sondes nucléiques issues de fragment d'ADN provenant exclusivement de C. *albicans* et **exclusivement** applicables à cette espèce de levure. Dans cette demande de brevet, on préconise de manière préférée la mise en oeuvre d'une sonde EOB2 constituée par tout ou partie d'une séquence nucléotidique, comprise dans le gène d'ADNr codant pour la sous-unité d'ARNr 5S. Cette séquence d'ADNr comprend 121 paires de bases localisées entre le nucléotide 937 et le nucléotide 1057 de la SEQ ID N0 : 1 du gène d'ADNr 5S, divulguée dans cette demande PCT. Secondairement, le WO-A-93 23 568 décrit une sonde EOB1 localisée dans la région intergénique non transcrite (IGR-NTS), située entre le gène d'ADNr codant pour l'ARNr 5S et le gène d'ADNr codant pour l'ARNr 18 S. Cette sonde EOB1 s'hybride avec une séquence d'ADNr comprise entre les nucléotides 1 et 936, de préférence 1 et 710 de la SEQ ID NO: 1.
Dans le cas où elles sont de faible longueur, ces sondes EOB2 et EOB1 peuvent être amplifiées par PCR amorces PS pA₁, PS pA₂, PCon₁, PCon₂.
La sonde EOB2 comprise dans l'ADNr 5S permet la détection des levures pathogènes, tandis que la sonde EOB1 est présentée comme étant interspécifique. En tout état de cause, il est clair que le WO-9323568 ne fait nullement mention de distinction infra-spécifique des souches des différentes espèces de levures citées (C. *albicans,* C. *krusei,* etc). La sonde EOB1 n'est pas une séquence source de stucture particulière, comme e.g. de type itérative comprenant plusieurs sous-séquences répétées homologues entre elles.
Le WO-93 23 568 ne divulgue donc pas, de structure génique typique d'identification et de détection inter et infraspécifique.

### BREVE DESCRIPTION DE L'INVENTION :

Dans cet état de la technique, l'un des objectifs essentiel de la présente invention est de fournir une sonde de détection et d'identification spécifique et/ou infraspécifique de levure, à vocation universelle dans le domaine des levures.

Un autre objectif essentiel de l'invention est de fournir une sonde, notamment nucléique, qui permette à la fois une détection spécifique et une identification infra spécifique de levure, de manière performante, fiable et reproductible.

Un autre objectif de l'invention est de fournir une sonde, notamment nucléique, qui soit de taille suffisante pour autoriser l'utilisation d'un marqueur, autre que radioactif et donc de mise en oeuvre plus commode.

Un autre objectif de l'invention est que cette sonde nucléique soit dirigée contre des séquences cibles d'ADN différent de celui codant pour des petites sous-unités 5,8 S ou 18 S d'ARN ribosomique, qui sont globalement peu représentatifs de l'espèce, car très conservées chez les micro-organismes.

Un autre objectif de l'invention est que cette sonde puisse être utilisée dans des milieux complexes biologiques du type sang humain, crachat, ou liquide céphalorachidien.

Un autre objectif essentiel de l'invention est de fournir une sonde notamment à *Candida krusei* qui permette de dépasser la simple détection d'espèces pour accéder à l'identification précise et au typage des levures au sein même de l'espèce *Candida krusei*.

Un autre objectif essentiel de l'invention est de fournir une sonde notamment à *Geotrichum candidum* qui permette de dépasser la simple détection d'espèces pour accéder à l'identification précise et au typage des levures au sein même de l'espèce *Geotrichum candidum.*

Un autre objectif essentiel de l'invention est de fournir une sonde de détection de levure qui soit composée d'outils génétiques du type séquence d'ADN et/ou d'ARN parfaitement identifiée et reproductible.

Un autre objectif essentiel de l'invention est de fournir une sonde de détection de levure qui soit significativement performante pour l'identification inter et infraspécifique d'un suffisamment grand nombre de levures, pour pouvoir être économique viable.

Un autre objectif essentiel de la présente invention est de fournir un procédé de détection et d'identification inter/infraspécifique de levures qui soit simple, non délicat et économique à mettre en oeuvre et dans lequel on a recours à la sonde susvisée.

Un autre objectif essentiel de l'invention est de fournir une sonde applicable notamment à la détection spécifique à l'identification infraspécifique de levure mais également comme marqueur épidémiologique, comme traceur de souches, ou dans le cadre d'une stratégie thérapeutique visant les affections à levure, entre autres.

Ces objectifs, et d'autres encore, sont atteints par la présente invention qui concerne une sonde, notamment, de détection spécifique et/ou infraspécifique de levures, du type de celle choisie parmi les outils génétiques (ou apparentés) suivants :
- au moins une partie :
   * d'au moins un fragment F d'acide nucléique ADN et/ou ARN recopié, construit et/ou isolé à partir d'au moins une séquence-source d'ADN ribosomal (ADNr) :
      → ne codant pas pour la synthèse d'ARN ribosomal (ARNr),
      → et localisée dans la région d'espacement intergénique (IGR) non transcrite (NTS) et comprise entre l'ADNr codant pour la sous-unité 25 S d'ARNr, et l'ADNr codant pour la sous-unité 18 S d'ARNr,
   * et/ou d'au moins un analogue Fa de ce fragment,
      résultant de la dégénérescence du code génétique ;
   * et/ou d'au moins un fragment Fc d'ADNc complémentaire du fragment F,
- au moins une partie des produits de transcription primaire du fragment F et/ou Fa et/ou Fc.
- et une association des outils énumérés ci-dessus.
caractérisé en ce que la séquence-source d'ADNr pour F et/ou Fa et/ou Fc est sélectionnée de telle sorte
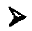 qu'elle soit itérative
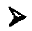 qu'elle comprenne au moins 2, de préférence de 2 à 12 sous-séquences répétées,
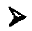 et que le degré d'homologie entre ces sous-séquences soit supérieur ou égal à 50 %, de préférence 55 %, et plus préférentiellement encore à 60 %.

### DESCRIPTION DETAILLEE DE L'INVENTION :

Il est donc du mérite des inventeurs cités dans la présente demande d'avoir pu isoler et caractériser un certain nombre de fragments F d'acide nucléique ayant une structure tout-à-fait particulière et qui *de facto*, constitue une véritable empreinte digitale non seulement d'une espèce donnée de levure mais également des différentes souches contenues dans cette espèce. Le mérite des inventeurs est d'autant plus grand que l'isolement et la caractérisation desdits fragments a été réalisé au sein des zones d'espacement intergénique non transcrites de cistrons ribosomaux, alors qu'il existait un courant scientifique, illustré notamment par l'article de BALEIRAS COUTO et al visé ci-dessus, selon lequel il semblait préférable et recommandé de s'intéresser aux régions transcrites des gènes ribosomaux pour la détection et l'identification de levures.

Il relève également du mérite des inventeurs d'avoir proposé des outils génétiques ou apparentés constitués par les susdits fragments pour la détection et l'identification inter-espèce de levures, de même que pour la mise en évidence infraspécifique (discrimination des levures d'une espèce donnée) et, en particulier, sans que cela ne soit limitatif, des espèces *Candida krusei* et *Geotrichum candidum*

Les fragments F, Fa, Fc d'acide nucléique selon l'invention ont pour caractéristique originale et avantageuse d'être non transcrits et de comprendre un ou plusieurs modules séquentiels de bases nucléiques (dénommés également séquences-sources itératives dans le présent exposé) comportant chacun une ou plusieurs sous-séquences répétées qui sont des copies très proches les unes des autres. C'est ou ce sont ces modules qui constituent un ou plusieurs motifs primaires utiles pour la détection/identification inter et infraspécifique d'une pluralité de levures.
Le nombre de bases nucléotidiques de ces modules ou séquences-sources varient dans une espèce à l'autre, mais on retrouve cette organisation similaire récurrente dans de multiples espèces de levures.
Sans vouloir être lié par la théorie, il semble que le moyen d'identification infraspécifique des sondes conformes à l'invention, puisse être fondé sur le polymorphisme qui, au sein d'une espèce donnée de levure, découle du nombre de sous-séquences dans les modules ou séquences-sources itératives, ou entrant dans la constitution des fragments F, Fa, Fc des sondes selon l'invention.

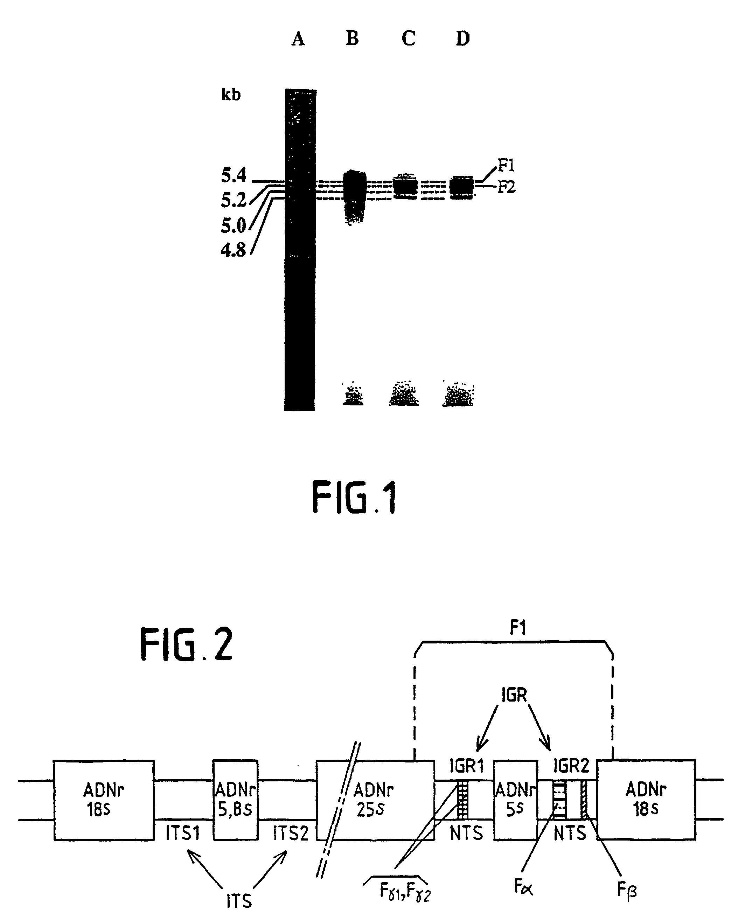

Suivant un premier mode de réalisation de l'invention, premier mode qui est d'ailleurs préféré, la séquence-source de F est localisée dans la région d'espacement intergénique 2 (IGR₂) non transcrite (NTS) et comprise entre l'ADNr codant pour la sous-unité 5S d'ARNr et l'ADNr codant pour la sous-unité 18 S d'ARNr (Cf **Fig.2** annexée).

Selon une variante avantageuse du premier mode de réalisation de l'invention, la séquence-source itérative de F correspond à une séquence Fα et comprend au moins trois sous-séquences répétées comportant chacune au moins 80, de préférence au moins 120 et plus préférentiellement encore entre 150 et 300 bases nucléotidiques.
En pratique, la séquence-source Fα comprend entre 3 et 10 sous-séquences répétées comprenant entre 150 et 250 bases nucléotidiques.

De préférence, les séquences-sources itératives de F et, en particulier Fα, de la sonde selon l'invention comprennent chacune au moins une sous-séquence de longueur tronquée par rapport aux autres sous-séquences, cette sous-séquence tronquée étant de préférence terminale.

S'agissant de la séquence-source itérative Fα, il est à considérer, sans pour autant vouloir être lié par la théorie, que le nombre de sous-séquences répétées élémentaires détermine le polymorphisme au sein d'une même espèce de levures. Cela pourrait résulter d'une réplication (duplication d'une ou plusieurs des sous-séquences répétées). Le tronc commun structurel entre les différentes souches d'une même espèce tient à une similitude dans l'organisation des répétitions de chaque sous-séquence.
Les phénomènes de réplication (duplication) à l'origine du polymorphisme entre souches sont liés à des recombinaisons, dont l'éventuelle séquence tronquée terminale évoquée ci-dessus, serait le reflet.

Toujours dans le cadre du mode préféré de mise en oeuvre de l'invention, selon lequel la séquence source F est dans l'IGR₂, il est prévu, conformément à une autre variante avantageuse, que la séquence-source itérative de F correspond à une séquence Fβ et comprend au moins trois sous-séquences répétées comportant chacune au moins 4, de préférence au moins 6 et, plus préférentiellement encore entre 7 et 25 bases nucléotidiques, chaque sous-séquence comprenant au moins un motif commun (**Com 1**) à toutes les sous-séquences et ayant une longueur inférieure ou égale à celle de la sous-séquence à laquelle il appartient.
En pratique, ces séquences-sources Fβ comprennent chacune 8 à 12 sous-séquences répétées comportant individuellement entre 7 et 13 bases nucléotidiques.

Conformément au deuxième mode de réalisation de la sonde selon l'invention, la séquence source de F :
- est localisée dans la région d'espacement intergénique 1 (IGR₁) non transcrite (NTS) et comprise entre l'ADNr codant pour la sous-unité 55 d'ARNr et l'ADNr codant pour la sous-unité 25 S d'ARNr
- et correspond à au moins une séquence Fγ et comprend au moins trois sous-séquences répétées comportant chacune au moins 4, de préférence au moins 5 et, plus préférentiellement encore entre 5 et 50, voire entre 5 et 45 et mieux encore entre 5 et 40 bases nucléotidiques, chaque sous-séquence comprenant au moins un motif commun (**Com 2** ou **3**) à toutes les sous-séquences et ayant une longueur inférieure ou égale à celle de la sous-séquence à laquelle il appartient.
un cas de figure particulièrement préféré étant celui dans lequel F correspond à au moins deux séquences Fγ : Fγ₁ et Fγ₂ ayant respectivement avantageusement de 5 à 20 et de 9 à 25 bases nucléotidiques.

Selon un autre de ces aspects, la présente invention concerne également des séquences d'ADN, de préférence ribosomique (ADNr), susceptibles de correspondre aux fragments F sus-évoqués : Fα, Fβ, Fγ, Fγ₁ et Fγ₂, ou susceptibles de contenir tout ou partie d'un ou plusieurs desdits fragments F noyés au sein d'une longue séquence nucléique, de préférence d'ADNr.

La présente invention a donc ainsi également pour objet une séquence d'ADNr caractérisée en ce qu'elle est choisie dans le groupe des séquences annexées suivantes : SEQ ID NO : 1 : ; SEQ ID NO : 2 : ; SEQ ID NO : 3 : ; SEQ ID NO : 4 : ; SEQ ID NO : 5 : ; les SEQ ID NO : 2 : à : 5 : étant particulièrement préférés.

En l'occurrence, la SEQ ID NO : 1 annexée contient toutes les SEQ ID NO 2 à 5 :
- SEQ ID NO : 2 : → base N 2256 à base 3512
- SEQ ID NO : 3 : → base N 4823 à base 4903
- SEQ ID NO : 4 : → base N 923 à base 1011
- SEQ ID NO : 5 : → base N 1012 à base 1151

Selon un exemple particulier d'illustration de l'invention, la sonde qu'elle concerne est caractérisé en ce que :
Fα correspond à SEQ ID NO : 2 :
Fβ correspond à SEQ ID NO : 3 :
Fγ₁ correspond à SEQ ID NO : 4 :
Fγ₂ correspond à SEQ ID NO : 5 :
Cet exemple de réalisation correspond à une souche de *Candida krusei*.

Suivant une disposition ou une modalité intéressante de l'invention, la sonde décrite ci-dessus est caractérisée
- en ce qu'elle comprend au moins une partie d'au moins l'un des fragments Fα, Fβ, Fγ₁, Fγ₂, le fragment Fα étant particulièrement préféré.
- en ce qu'elle est appliquée :
   ▷ à la détection/identification spécifique et infraspécifique de levures,
   ▷ ou comme marqueur épidémiologique,
   ▷ ou encore comme traceur de souche,
   ▷ ou bien enfin dans le cadre d'une stratégie thérapeutique visant des affections à levures.

S'agissant de l'origine des séquences d'acides nucléiques typiques, conformes à l'invention, on a vu qu'elles sont naturelles puisqu'issues à la base du génôme de souches de levures.

Mais il va de soi qu'il est tout à fait à la portée de l'homme du métier, de préparer des séquences nucléiques « non naturelles » en recopiant les séquences par exemple selon les principes du clonage ou de la réaction de polymérisation en chaîne (PCR), ou par voie de synthèse. Notamment, on peut synthétiser des séquences nucléiques à l'aide d'appareils de synthèse automatiques, tels que ceux commercialisés par Applied Biosystems.

Aussi, dans tout le présent exposé les termes fragments ou séquences nucléiques, désignent aussi bien ceux de type synthétique, que ceux de type naturel.

Dans le cadre de l'application en détection / identification, les fragments F, Fa et Fc sont utilisés comme sonde selon le principe d'hybridation ADN-ADN ou ADN-ARN. Une sonde constituée de tels fragments permet la réalisation de tests de détection et d'identification rapides, sensibles, et inter/infraspécifiques d'une pluralité d'espèces de levure. En d'autres termes, cette sonde peut notamment constituer un excellent marqueur de typage des souches d'une espèce donnée et en particulier de *Candida krusei* ou *Geotrichum candidum*, ces dernières espèces ayant été sélectionnées, parmi d'autres, pour servir de support à l'exemplification du présent exposé.

Il faut considérer que l'hybridation de la sonde nucléique de l'invention avec des séquences cibles d'acide nucléique de levure (e.g. *C. krusei*), peut se faire à l'aide de la totalité des fragments F visés ci-dessus mais aussi avec seulement une fraction de tout ou partie de ces fragments, dont la taille reste, de préférence, supérieure ou égale à 5 Bases, de préférence à 10 Bases et, plus préférentiellement encore à 100 Bases.

Une autre des caractéristiques avantageuses de la sonde nucléique selon l'invention est liée au fait que cette dernière peut être marquée à l'aide de moyens de marquage aptes à révéler l'hybridation. Ces moyens de marquage peuvent être radioactifs ou non. Parmi les marqueurs radioactifs classiques, on peut citer le P³². S'agissant des marqueurs non radioactifs, on peut mentionner à titre d'exemples, les enzymes fixés tels que la peroxydase.

Par ailleurs, la sonde selon l'invention peut éventuellement être employée comme source d'amorces spécifiques utilisables dans des réactions de polymérisation en chaîne (PCR). Ces amorces constituent un autre objet de l'invention.

Comme cela a déjà été signalé, la sensibilité de la sonde selon l'invention est excellente, de sorte qu'elle est parfaitement adaptée à la détection et/ou au typage de levures (e.g. *Candida krusei* ou *Geotrichum candidum*) par hybridation en "dot-blot" ou en "southern-blot". Cette sensibilité se vérifie dans des essais indirects d'hybridation (méthode des dépôts, dot-blot, Southern-blot, Northern-blot, technique d'hybridation « sandwich ») mais aussi dans des expériences d'hybridation directe in situ. Ces dernières consistent à faire une empreinte sur filtre, de colonies en croissance sur un milieu de culture solide et à amener la sonde d'hybridation directement sur le filtre.

Comme cela ressort du texte supra ainsi que de la revendication 1, la sonde selon l'invention est de préférence de nature nucléique, c'est-à-dire constituée d'acides nucléiques du type ADN, ADNc, ARNm ou leurs variants et analogues. Ces acides peuvent être d'origine naturelle ou synthétique.

Il est intéressant de noter que la sonde selon l'invention peut être employée efficacement dans tout milieu complexe, comme peuvent l'être les milieux biologiques : sang, crachat, liquide céphalorachidien, etc. Cela n'interfère nullement sur ses qualités de détection et d'identification.

La présente invention concerne également un procédé de détection et d'identification inter et/ou infraspécifique de levure caractérisé en ce qu'il consiste à mettre en oeuvre au moins une sonde nucléique telle que décrite ci-dessus.
Ce procédé s'inscrit dans le cadre des méthodologies connues dans le domaine de la détection et de l'identification génétique des microorganismes.
De préférence, ce procédé comprend les étapes suivantes :
- on extrait l'ADN total génomique des souches à étudier,
- on soumet éventuellement cet ADN total à une digestion enzymatique à l'aide d'au moins une enzyme de restriction,
- on dénature l'ADN total éventuellement digéré,
- on met en présence l'ADN total ainsi dénaturé, avec la sonde dotée d'au moins un marqueur, de façon à réaliser l'hybridation,
- on élimine l'ADN et la sonde non hybridée,
- et on révèle l'hybridation à l'aide du marqueur.
Il s'agit d'une technique d'hybridation dans laquelle l'ADN cible du micro-organisme à étudier, est soumis à une dénaturation, avec ou sans digestion enzymatique préalable. L'étape qui suit est celle de réassociation des brins séparés d'ADN dénaturé avec la sonde pour reformer des appariements de paires de base originaux.
Lors de cette étape de réassociation, les molécules simple brin de la sonde et de la cible sont mises en condition plus ou moins favorables pour l'appariement (plus ou moins stringentes). Dans des conditions très stringentes seules les molécules dont les séquences sont complémentaires pour un grand nombre de bases, s'hybrident pour former une molécule double brin. La sonde est alors spécifique de la cible.
Avec une sonde marquée à l'aide d'un élément radioactif, tel que le P³², ou d'une enzyme greffée comme par exemple la peroxydase, l'hybridation est aisément révélée qualitativement et quantitativement.

Dans le cas où la sonde comprend des fragments (séquences) nucléiques typiques d'origine naturelle, ceux-ci sont naturellement dénaturés à l'instar des cibles qu'ils sont susceptibles de détecter. Ce n'est évidemment pas le cas pour des fragments synthétiques.

La présente invention vise également un réactif de détection/identification de levures comprenant au moins une sonde selon l'invention.

### APPLICATION INDUSTRIELLE

Il ressort de de ce qui précède que la sonde nucléique selon l'invention ainsi que le procédé en faisant application, sont parfaitement spécifiques des levures (e.g. *Candida krusei*. ou *Geotrichum candidum*) et offrent une excellente sensibilité. Cela leur ouvre des débouchés tout à fait intéressants dans les domaines applicatifs de l'identification et du criblage différenciation des souches de différentes espèces. (diagnostic médical, contrôles industriels en alimentaire, fermentation...).

La sonde et le procédé selon l'invention intéressent diverses espèces de levure et, en particulier, celles d'intérêt médical telles que celles du genre Candida ou d'intérêt industriel (agroalimentaire tel que *Geotrichum Candidum*).

Outre, la détection qui regroupe l'identification et le typage de microorganisme de type levure, la sonde selon l'invention pourrait être appliquée dans le cadre d'une stratégie thérapeutique dirigée à l'encontre des affections à levures. Plus précisément, cela signifie que les fragments F formant la sonde peuvent être employés comme cibles à atteindre, pour des principes médicamenteux actifs contre les levures. Ce rôle de cible pour médicament pourrait notamment être tenu par l'ADN ou par ses produits de transcription primaire.

L'emploi de la sonde selon l'invention comme marqueur épidémiologique ou comme traceur de souches constituent d'autres variantes d'applications de la sonde selon l'invention.

L'illustration de la présente invention effectuée ci-après sur la base de travaux réalisés pour les espèces de levures *Candida krusei* et *Geotrichum candidum*, mais il va de soi que les exemples basés sur *Candida krusei* et *Geotrichum candidum* pourront être extrapolés aux autres espèces de levures et, notamment à celles d'intérêt industriel et médical.

En tout état de cause, l'invention sera mieux comprise, d'autres de ses avantages et variantes de réalisation ressortiront bien, à la lumière des exemples non limitatifs qui suivent et qui décrivent en référence aux dessins annexés la structure d'une sonde selon l'invention et en particulier à des fragments F qui la constitue, ainsi que plusieurs procédures de détection et d'identification en faisant application.

### DESCRIPTION DES FIGURES :

- la liste des séquences annexée donne la structure des SEQ ID : 1 à 5 : correspondant respectivement aux fragments F1 et Fα, Fβ, Fγ₁ et Fγ₂.

Le fragment F1 donne le séquençage de la région intergénique d'espacement (IGR) entre l'ADNr 25S et l'ADNr 18 S d'un cistron ribosomal de *Candida krusei*.
Les exemples de fragment Fα, Fβ, Fγ₁ et Fγ₂ seront également dénommés respectivement CKRS1/b/a1/ a2, dans ce qui suit.
- la **Figure 1** annexée représente l'hybridation Southern-blot de *Candida krusei* LMCK31 par des sondes F1 et/ou F2.
- la **Figure 2** est une représentation schématique d'un cistron d'ADN ribosomal (ADNr) montrant la localisation du fragment F1 dans la région d'espacement intergénique (IGR) et, au sein de cette dernière, l'existence des fragments Fα, Fβ, (IGR₂) de même que Fγ₁ et Fγ₂ (IGR₁), conformes à l'invention.
- la **Figure 3** est une carte de restriction du fragment F1 de la **Figure 2**.
- la **Figure 4** est une représentation schématique de la stratégie de séquençage, en corrélation avec le fragment F1 de la **figure 3.**
- la **Figure 5** est une représentation schématique éclatée du fragment Fα montré aux **Figures 2** et **3.**
- la **Figure 6** donne le séquençage de Fα, en particulier de ses sous-séquences Kre-0 à 7, en faisant une comparaison entre les différentes sous-séquences. Des étoiles représentent des bases analogues à celles de la sous-séquence Kre-1. >>ivr1<< est une séquence inversée comprenant 8 bases. >>pal1<< est un Palindrome de 8 bases.
- Les **Figures 7A, 7B** et **7C** sont des représentations comparées des sous-séquences formant les séquences-sources itératives Fβ, Fγ₁ et Fγ₂ respectivement.
- les **Figures 8A** et **8B** sont des éléctrophorèses à champ pulsé de chromosomes de *C. krusei* et de *C. albicans*, respectivement avant et après hybridation avec des sondes F1,
   Fα et Fβ, Fγ₁ et Fγ₂.
- la **Figure 9A** est une photographie d'une séparation par électrophorèse sur gel d'agarose, de fragments d'ADN de souches de différentes espèces de levures (1 à 11), digérés par *Eco*RI.
- la **Figure 9B** est une hybridation selon Southern d'ADN de différentes espèces de la **Figure 9A**, illustrant la spécificité de la séquence-source itérative Fα (ou CKRS-1) utilisée comme sonde.
- la **Figure 10A** est une photographie du gel d'électrophorèse de l'ADN de plusieurs souches de l'espèce *Candida krusei*.
- la **Figure 10B** est une hybridation selon Southern de l'ADN de différentes souches de *C. krusei*, illustrant le polymophisme mis en évidence lorsque la séquence Fα (ou CKRS-1) est utilisée comme sonde.
- la **Figure 11** montre le résultat de l'amplification sélective en PCR de la région comprenant Fα.
- la **Figure 12** est une représentation schématique de la Carte de restriction du Fragment F₁₀ forment la sonde A₂O₂₇ de *Geotrichum candidum*. Cette sonde comprenant un sous-fragment F dont la séquence-source itérative est **F**δ (région variable GCRS-A).
- la **Figure 13** représente un profil d'hybridation (Southern) avec la sonde A₂O₂₇ spécifique de *Geotrichum candidum* et développée selon le mode de la sonde F(Fα, F β, Fγ₁, Fγ₂) de *Candida krusei*.

### EXEMPLES

### EXEMPLE I : SEQUENÇAGE D'UN FRAGMENT F1 D'ADNr DE C. KRUSEI PROVENANT DE LA DIGESTION DE LA SOUCHE LMCK1 PAR ECORI

1.1. La souche LMCK 31 a été isolée dans un prélèvement broncho-alvéolaire chez un patient hospitalisé. Cette souche a été déposée le 22 Octobre 1993 à la CNCM de l'Institut Pasteur de PARIS sous la référence **I-1372.**
Cette souche LMCK 31 est cultivée sur des milieux de culture du type gel AGAR YM (1% glucose, 0,3 % extrait levure, 0,3 % extrait de malt, 0,5 % de pactopeptone) ou AGAR SM (1% glucose, 1% bactopeptone, 0,1 % MGSO₄. 7H₂O, 0,22 % KH₂PO₄, 0,1 % K2 HPO₄, 0,1 % extrait levure).

### 1.2. Isolement et clonage d'un fragment F1

Un fragment F1 de longueur approximativement égale à 5,4 kb est isolé à partir d'ADN génomique de la souche LMCK31, conformément au protocole décrit dans la demande de brevet PCT WO-95 11 991. Le fragment F1 a été sélectionné parmi la multitude de fragments produits par la digestion de l'ADN total de LMCK31 par *Eco*RI ou par *Hin*fI.
Plus précisément on a réalisé une hybridation "southern blot" en mettant en oeuvre 5 µg d'ADN génomique de *Candida krusei* LMCK31 digéré soit par *Eco*RI ou *Hin*fI puis séparé par électrophorèse selon leur taille sur un gel d'agarose (0,8 % m/v). L'ADN séparé est dénaturé dans le gel par une solution de soude puis neutralisé par un tampon tris. L'ADN est ensuite transféré sur une membrane en nylon (Amersham life science inc., ARLINGTON HEIGHTS, IL) puis hybridé avec la sonde contenant des fragments F1 et F2 correspondant à l'IGR1 et 2 d'ADNr ou avec des plasmides contenant, soit le fragment F1, soit le fragment F2. La révélation de l'hybridation par "southern-blot" est effectuée à l'aide du kit ECL d'Amersham. Les bandes d'hybridation sont obtenues par marquage avec la péroxydase et révélation par chimiluminescence.
La **Figure 1** annexée donne les résultats obtenus. La colonne A correspond aux produits de la digestion par *Eco*RI de l'ADN de *Candida krusei* non hybridé avec une sonde. La colonne B correspond à l'hybridation avec une sonde comprenant des fragments F1 et F2. La colonne C correspond à une hybridation avec une sonde plasmidique contenant F1 et la colonne D à l'hybridation avec une sonde plasmidique contenant F2.
Les poids moléculaires estimés des quatre bandes d'hybridation des colonnes B, C, D sont données en kilobase (kb) en marge gauche.

### 1.3. Séquençage de F1

F1 est séquencé dans son intégralité dans les deux directions. La méthode utilisée pour ce séquençage est une méthode classique dans le domaine technique considéré. Il s'agit de la méthode par les terminaisons DIDEOXY utilisant la séquénase version 2.0. (US biochemical, cleveland OH). Les moyens utilisés sont le kit ERASE-A-base (Promega - Madison, WI).
La **Figure 2** montre de manière schématique la localisation du fragment F1 dans le cistron d'ADN ribosomal de LMCK 31.

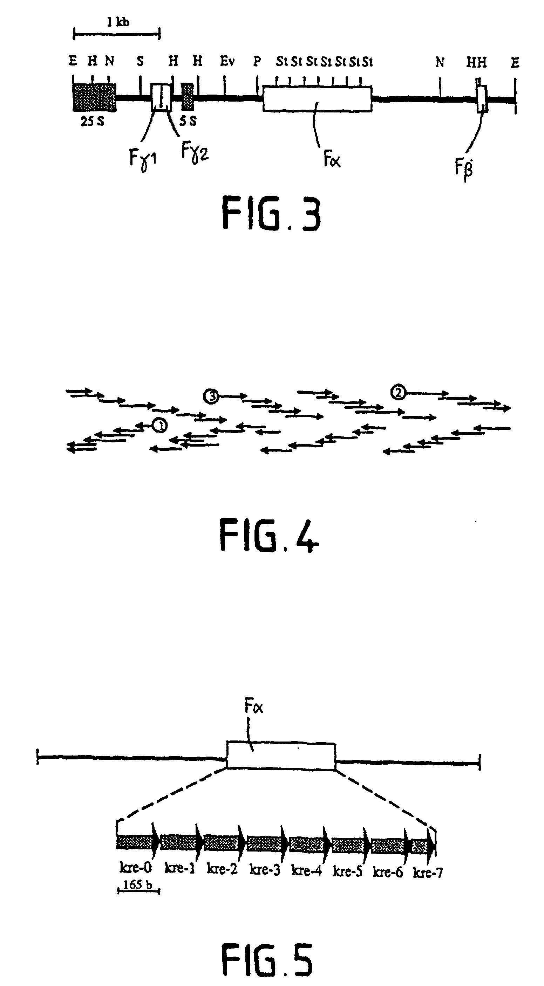

La **Figure 3** annexée donne la carte de restriction du fragment F1. Les abbréviations utilisées pour les enzymes de restriction ont les significations suivantes : E = *Eco*RI (0,5261), Ev = *Eco*RV (1803), H = *Hin*fI (229, 1155; 1551, 4828, 4864), N = *Nsi*I (401, 4383), P *= Pst*I (2183), S = *Sma*I (803); St = *Sty*I (2425, 2589, 2754, 2919, 3084, 3249, 3414).
La **Figure 4** annexée donne la stratégie de séquençage adoptée. Dans cette **Figure 4**, les chiffres encerclés 1, 2, 3 correspondent à des sous-clones produits de déletions.
Les chiffres donnés entre parenthèses après les enzymes correspondent au numéro de base nucléotidique identifiant le site de coupure.
Il apparaît donc que F1 est une macro-séquence comprenant 5261 paires de base et ayant un taux en bases GC de 42,2 % molaire. La séquence complète de F1 est donnée par SEQ ID NO : 1 : dans la liste de séquence annexée. F1 comprend :
- 488 paires de base sur l'extrémité 3' de l'ADNr 25 S,
- la région d'espacement intergénique 1 (IGR₁),
- l'ADNr 5 S
- et la région d'espacement intergénique 2 (IGR₂).
Comme, cela ressort des **Figures 2** et **3**, l'IGR₂ (ou NTS = séquence non transcrite) comprend deux séquences sources itératives Fα et Fβ. Tandis que l'IGR₁ renferme les deux séquences-sources itératives Fγ1 et Fγ2.

### 1.3.1. Fα

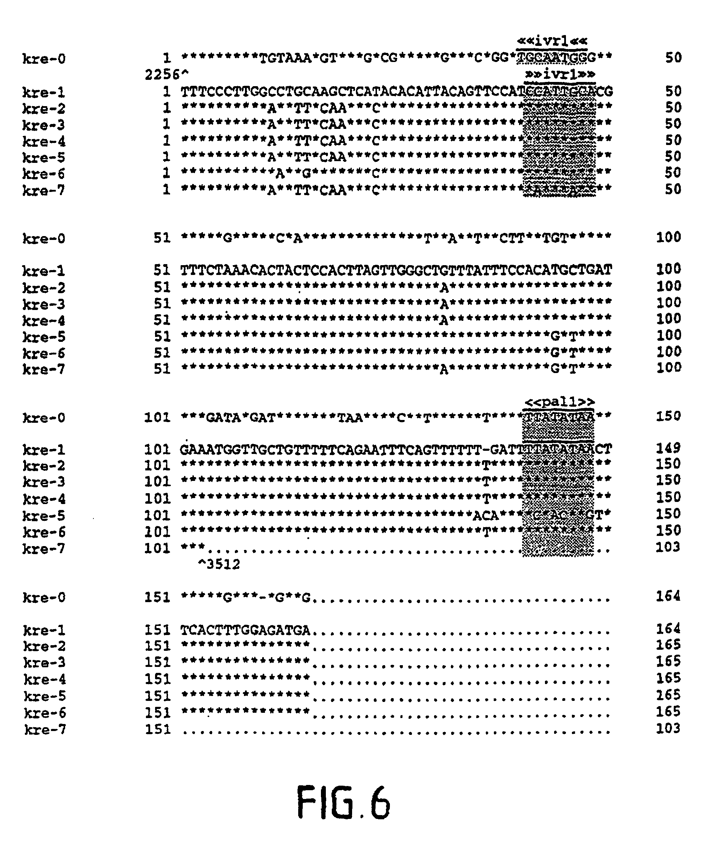

La séquence-source itérative Fα a une longueur de 1256 paires de base. (base 2256 à base 3512). Fα est représentée schématiquement sur la **Figure 5**. La **Figure 6** et la SEQ ID NO : 2 : de la liste de séquences annexée, donnent le séquençage complet de Fα.
Fα est constitué de 8 sous-séquences répétées désignées par Kre-0 à Kre-7 respectivement. Les 7 premières sous-séquences répétées Kre-0 à Kre-6 ont une longueur variant entre 164 et 165 paires de base. La dernière sous-séquence Kre-7 comporte 103 paires de bases (voir **Figures 5** et **6**).
Le fragment d'ADN correspondant à Fα à une teneur en bases GC de 35 % molaire.

### 1.3.2. Fβ

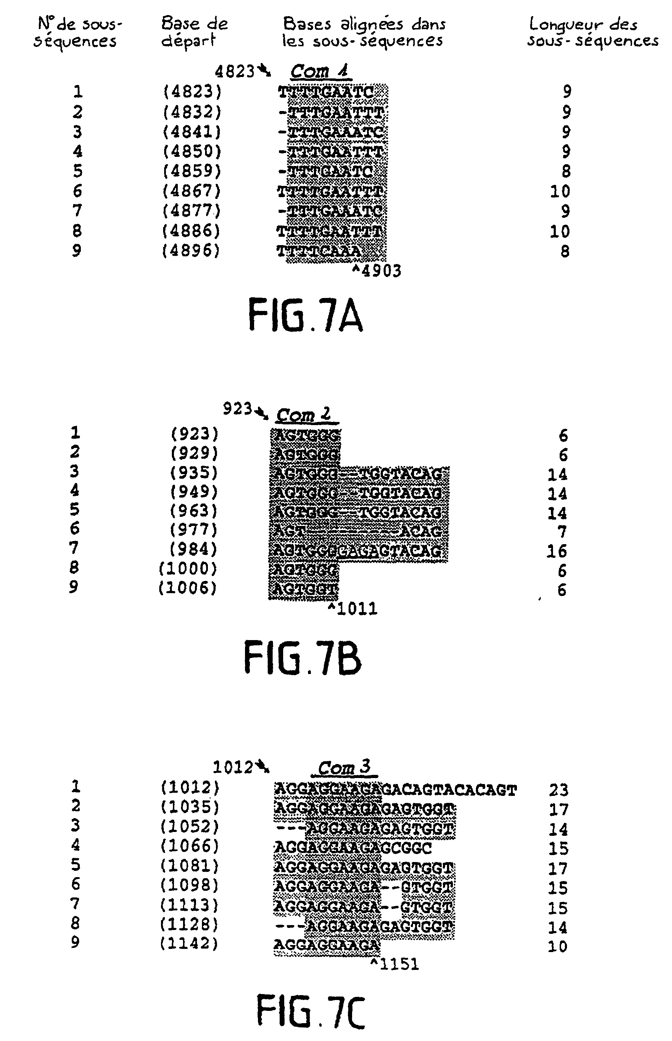

L'autre séquence-source itérative d'un fragment F susceptible d'entrer dans la constitution d'une sonde selon l'invention, est compris dans l'IGR₂ et désigné par la référénce Fβ. Le séquençage de Fβ est représenté sur la **Figure 7A** et sur la SEQ ID : NO : 3 : de la liste de séquences annexée. La séquence-source Fβ comprend 9 sous-séquences répétées comprises entre la base 4823 et la base 4903. Ces sous-séquences ont une longueur variable entre 8 et 10 bases. Elles contiennent un noyau commun de 6 bases constituant un motif commun (référencé sur la **Fig. 7A** par **Com 1**), répété soit directement (les uns à la suite des autres) soit en étant séparés par des espacements de quelques bases nucléotidiques.

### 1.3.3. Fγ1 et Fγ2

Ces deux séquences-sources itératives Fγ1 et Fγ2 sont localisées dans l'IGR₁. Fγ1 comprend 9 sous-séquences répétées de longueur variant entre 6 et 16 bases, ces sous-séquences s'étalant de la base 923 à la base 1011 (**Figure 7B**). Fγ2 s'étend de la base 1012 à la base 1151 et comporte 9 sous-séquences de longueur variant entre 10 et 17 bases (**Figure 7C**). Les sous-séquences de Fγ2 sont des copies très proches les unes des autres. Deux noyaux aux motifs communs de 6 et 7 bases peuvent être isolés respectivement dans Fγ1 et Fγ2. Les motifs communs de Fγ1 et Fγ2 sont identifiés par **Com 2** et **Com 3** sur les **Figures 7B** et **7C** respectivement.

A l'instar de **Com 1** de Fγ, **Com 2** et **Com 3** sont répétés soit directement (les uns à la suite des autres) soit en étant séparés par des espacements de quelques bases nucléotidiques.

### EXEMPLE 2 : ETUDE DE L'HOMOLOGIE ENTRE LES HUIT SOUS-SEQUENCES DE Fα

L'homologie des sous-séquences Kre-0 à Kre-7 a été étudié en comportant les sous-séquences après alignement optimal.
Les résultats obtenus sont donnés dans le tableau I qui suit :

**TABLEAU I**

| *POURCENTAGE DE BASES QUI "ALLUMENT" OU QUI CORRESPONDENT ENTRE LES 8 SOUS-SEQUENCES **Kre-0 A Kre-7*** | | | | | | | | |
|---|---|---|---|---|---|---|---|---|
| % de bases correspondantes (no. des bases différentes) | | | | | | | | |
| Séquences | Kre-0 | Kre-1 | Kre-2 | Kre-3 | Kre-4 | Kre-5 | Kre-6 | Kre-7* |
| kre-0 | 100 | | | | | | | |
| Kre-1 | 71 (47) | 100 | | | | | | |
| Kre-2 | 77(39) | 95(9) | 100 | | | | | |
| Kre-3 | 77(39) | 95(9) | 100 | 100 | | | | |
| Kre-4 | 77(39) | 95(9) | 100 | 100 | 100 | | | |
| Kre-5 | 67(56) | 90(17) | 93(11) | 93(11) | 93(11) | 100 | | |
| Kre-6 | 71(47) | 96(6) | 94(10) | 94(10) | 94(10) | 91(12) | 100 | |
| Kre-7* | 66(35) | 88(12) | 96(4) | 96(4) | 96(4) | 97(3) | 90(10) | 100 |

| | | | | | | | | |
|---|---|---|---|---|---|---|---|---|
| * Les 103 bases de Kre-7 ont été comparées avec les 103 premières bases de Kre-n = 0 à 6. | | | | | | | | |

### EXEMPLE 3 : HYBRIDATION DE CHROMOSOMES DE CANDIDA KRUSEI ET DE CANDIDA ALBICANS AVEC DES SONDES COMPRENANT LES FRAGMENTS F1 OU Fα ET Fβ OU Fγ1, Fγ2

Cet exemple vise à démontrer que la localisation des fragments F1, Fα, Fβ Fγ1 et Fγ2 constituant la sonde selon l'invention est seulement sur les chromosomes contenant des cistrons d'ARNr.

**3.1.** Les souches mises en oeuvre sont LMCK31 pour *Candida krusei* et la souche 3153A pour *Candida albicans*.

**3.2.** Les fragments F1, Fα, Fβ Fγ1 et Fγ2 mis en oeuvre sont ceux isolés dans les Exemples 1 et 2.

**3.3.** La technique mise en oeuvre est celle d'électrophorèse transversale à champ pulsé. Des plaques d'agarose contenant l'ADN chromosomique sont préparées à partir de suspensions de culture de souche *C. krusei* LMCK 31 et *C. albicans* 31 53A. Les cellules sont converties en sphéroplastes par la méthode de shareman et al, selon laquelle on met en oeuvre la ZYMOLYASE 20 T. Les sphéroplastes sont tout d'abord lavés deux fois dans une solution de sorbitol molaire puis remis en suspension à raison de 10⁹ cellules/ml. Les sphéroplastes sont mélangées avec un volume = d'agarose seaplaque GT à 1 % (FMC bioproducts, Rockland ME) et immédiatement versé dans un moule. Les plaques sont traitées avec du SARCOSYL à 1% et avec une protéinase K titrant 100 mg/ml et enfin incubé à 50° pendant 60 heures. Les plaques sont ensuite lavées dans une solution tampon TE (10 mM tris-HCL), ph 8, 1mM EDTA) et stockée dans ce milieu à 4° C. Les ADN chromosomiques sont séparés par électrophorèse sur gel à champ pulsé dans un dispositif Gene line (BECKMAN, FULLERTON, CA) en utilisant de l'agarose LE 0,65 % (FMC bioproducts, Rockland me) ainsi qu'un tampon tris - acétate 0,04 M tris acétate, 0,001 M EDTA pH8). L'électrophorèse est menée selon les cinq étapes telles que décrites ci-après : 65 V pendant 6 heures avec une durée de commutation d'une minute ; 65 V pendant 12 heures avec une durée de commutation de 2 mn ; 65 V pendant 16 heures avec une durée de commutation de 4 mn ; 65 V pendant 20 heures avec une durée de commutation de 7 mn et 65 V pendant 18 heures avec une durée de commutation de 10 mn. Les gels sont ensuite révélés avec du bromure d'ethidium, photographiés puis transférés sur une membrane de nylon ZETABIND. L'hybridation et le lavage sont réalisés selon la méthodologie de CHURCH et GILBERT. Les bandes chromosomiques sont sondées avec les différentes sondes marquées radioactivement : F1, Fα et Fβ Fγ1 et Fγ2, selon la méthode d'hybridation sur membrane.

### 3.4. Résultats

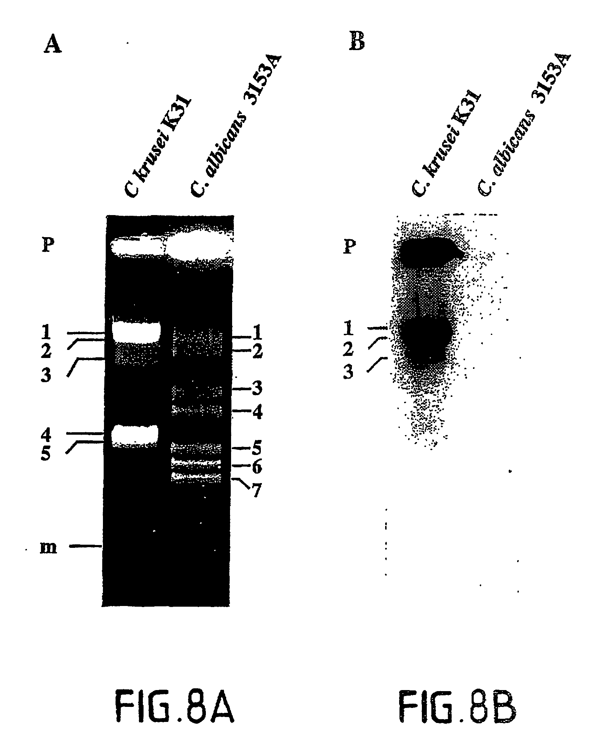

La **Figure 8A** montre les gels d'électrophorèse à champ pulsé qui montre que l'on a séparé 5 chromosomes majeurs pour *Candida krusei* et 7 chromosomes majeurs pour *Candida albicans*.
La **Figure 8 B** montre que les sondes F1, Fα et Fβ, Fγ1 et Fγ2 hybrident uniquement avec les trois plus gros chromosomes 1, 2, 3 de *Candida krusei*. Les sondes n'hybrident avec aucun des 7 chromosomes de *Candida albicans*. Ce qui démontre la spécificité inter-espèces desdites sondes. La **Figure 8A** montre également un minichromosome m (pour *Candida krusei*).
La **Figure 8** montre une hybridation sur membrane dans laquelle les sondes ont été mises en contact avec des chromosomes pendant 4 jours.
Le code P sur les **Figures 8A** à **8B** correspond à la zone de dépôt de l'échantillon.

### EXEMPLE 4 : TEST DE DETECTION ET D'IDENTIFICATION ENTRE ESPECES

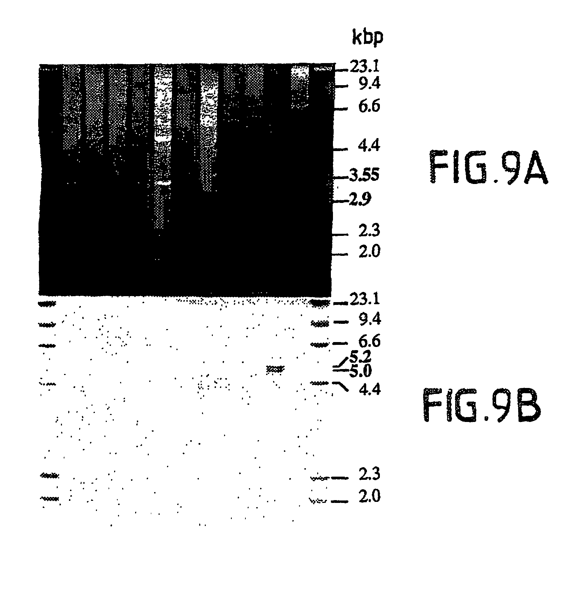

La **Figure 9A** montre le gel d'électrophorèse de l'ADN de souches de différentes espèces de levures, digéré par l'enzyme *Eco*RI. La **Figure 9B** montre l'hybridation selon Southern correspondante lorsque les sondes F1, Fα et Fβ, Fγ1 et Fγ2 sont utilisées, en étant marquées à la peroxydase. Le protocole experimental est le même que celui décrit décrit dans l'Exemple 1 supra ainsi que dans l'Exemple 3 du **WO-95/11991.** Les sondes hybrident uniquement avec l'ADN de *C. krusei* (CBS 573T, bande n° 10) et pas avec l'ADN des autres levures testées. Ceci démontre la spécificité interespèce desdites séquences.
Dans les essais correspondant aux **Fig. 9A** et **9B**, les microorganismes considérés sont les suivants :
S : Standard : Phage lambda digéré par *Hind*III
1 : *C. tropicalis* CBS 94T
2 : *C. parapsilosis* CBS 604T
3 : *C. guilliermondii* CBS 6021T
4 : *C. lusitaniae* CBS 6936T
5 : *C. kefyr* CBS 607T
6 : *C. albicans* serotype B
7 : *C. albicans* serotype A
8: *C. Albicans* ATCC 2091
9 : *C. valida* CBS 638T
**10 : *C. Krusei* CBS 573 T**
11 : *Y. lipolytica* CBS 6124 T
S : Standard : Phase lambda digéré par *Hind*III

### EXEMPLE 5 : TEST DE TYPAGE INFRA-SPECIFIQUE POUR DIFFERENTES SOUCHES DE C. Krusei

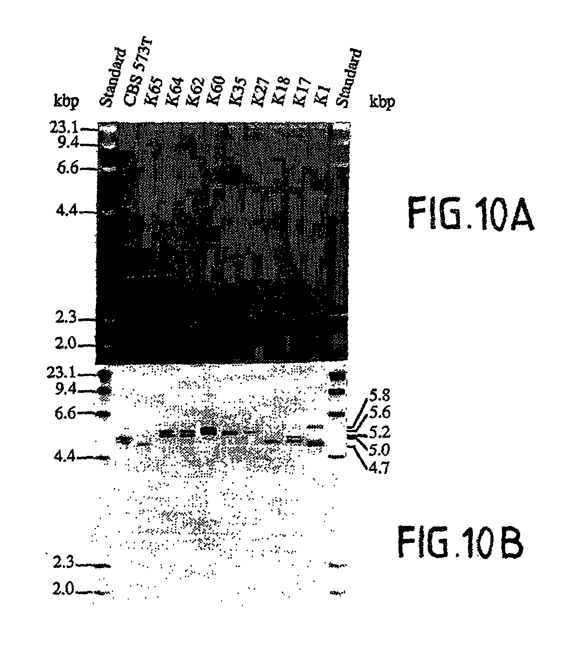

La **Figure 10A** montre le gel d'electrophorèse de l'ADN de plusieurs souches de l'espèce *C. Krusei*, digéré pour l'enzyme *Eco*RI. La **Figure 10B** montre l'hybridation selon Southern correspondante lorsque les sondes F1, Fα et Fβ, Fγ1 et Fγ2 sont utilisées, marquées à la peroxydase. Le protocole experimental est le même que celui décrit dans l'Exemple 1 supra. (ou Exemple 3 du **WO 95/11991**). Les sondes hybrident avec toutes les souches de *C. krusei* testées, ce qui illustre leur intérêt pour la détection et l'identification de cette espèce. En outre, ces sondes révèlent un polymorphisme de la taille des fragments de restriction selon les sondes. Ceci illustre l'intérêt de ces sondes pour différencier les souches entre-elles au niveau infraspécifique et donc pour le typage des souches dans le cadre d'études épidémiologiques ou le traçage des souches en milieu industriel. Toujours à titre d'exemple les souches référencées K62-K64 dont les profils d'hybridation sont identiques, proviennent d'un même patient.

### EXEMPLE 6 : TEST D'IDENTIFICATION ET TYPAGE DE SOUCHES DE C. KRUSEI PAR REACTION DE POLYMERISATION EN CHAINE (PCR) DE LA REGION Fα (OU CKRS-1)

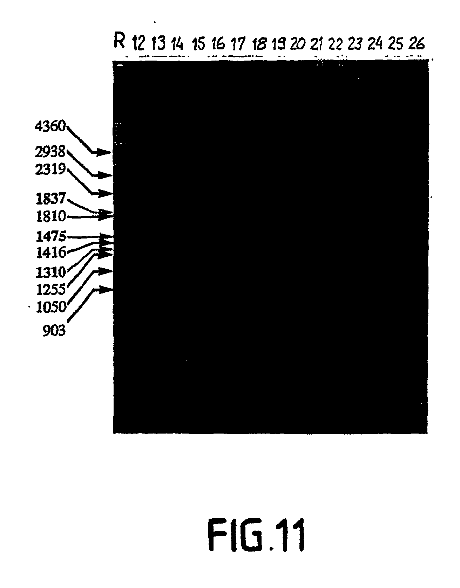

La **Figure 11** montre le résultat de l'amplification sélective (selon la méthode PCR) de la région Fα (CKRS-1) à l'aide des amorces Arnol : CGTAGGATACTAACCACAGC et Arno 2 : GGCCAACACATACATACCTT dont les séquences sont choisies dans le fragment F1 de *C. krusei* (positions 2138-2167 et 3989-3970). Les conditions d'amplification sont celles décrites par Carlotti et al. 1994: Systematic and Applied Microbiology 17, 380-386. La procédure d'amplification par PCR est la suivante : 50 µl de la solution d'ADN à tester (ADN de *C. krusei* et ADNs d'autres espèces de levures) contenant environ 10 à 100 pg sont ajoutés à 50 µl du mélange réactionnel contenant la Taq polymérase dans un tube eppendorf et placé sur un thermocycleur. La dénaturation initiale dure 3 minutes à 92° C. Elle est suivie de 30 cycles constitués de : 1 minute à 92°C, 1 minute à 60°C, 2 minutes à 72° C. L'extension finale dure 10 minutes à 72° C.
Les différentes souches testées dans cet exemple sont désignés par les références R, 12 à 26 sur la **Figure 11**. La légende de ces références est donné ci-après :
R : Raoul
12: *C. krusei* CBS 573T
13 : *C. krusei* LMCK 31
14 : controle Négatif
15 : *S. cerevisiae* CBS 1171
16 : *C. albicans* ATCC 2091
17 : *C. norvegensis* LM Q243
18 : *C. glabrata* CBS 138T
19 : *Y. lipolytica* CBS 6124T
20 : *C. kefyr* CBS 607T
21 : *C. lusitaniae* CBS 6936T
22 : *C. guilliermondii* CBS 6021T
23 : *C. parapsilosis* CBS 604T
24 : *C. rugosa* SIPH
25 : *C*. *valida* CBS 638T
26 : *C. inconspicua* CBS 180T
Du fait de la spécificité de la région (CKRS-1) cible et des amorces choisies, seuls les ADNs des souches de *C. krusei* (réf. 12, 13) ont donné un signal d'amplification positif (**Figure 11**). Les ADNs des autres espèces de levures testées n'ont pas été amplifiés. En outre, les différences de la taille des fragments amplifiés chez les deux souches de *C. krusei* testées traduisent le polymorphisme de la région Fα(CKRS-1). Cet exemple illustre bien l'intérêt de tests d'amplification par PCR de la région Fα (CKRS-1) pour l'identification des souches de *C. krusei* et pour leur typage simultané. Cette méthodologie présente l'avantage d'être très rapide et utilisable directement sur les prélèvements biologiques (crachats, sang, selles, LCR) ou alimentaires.

### EXEMPLE 7 : TEST DE DETECTION, D'IDENTIFICATION-TYPRAGE DES LEVURES DE L'ESPECE Geotrichum candidum

**7.1.** L'isolement et le clonage du Fragment F₁₀ comprenant Fδ compris dans la sonde A₂O₂₇ développée pour *Geotrichum candidum*, ont été réalisés conformément au protocole décrit à l'exemple 1 supra.

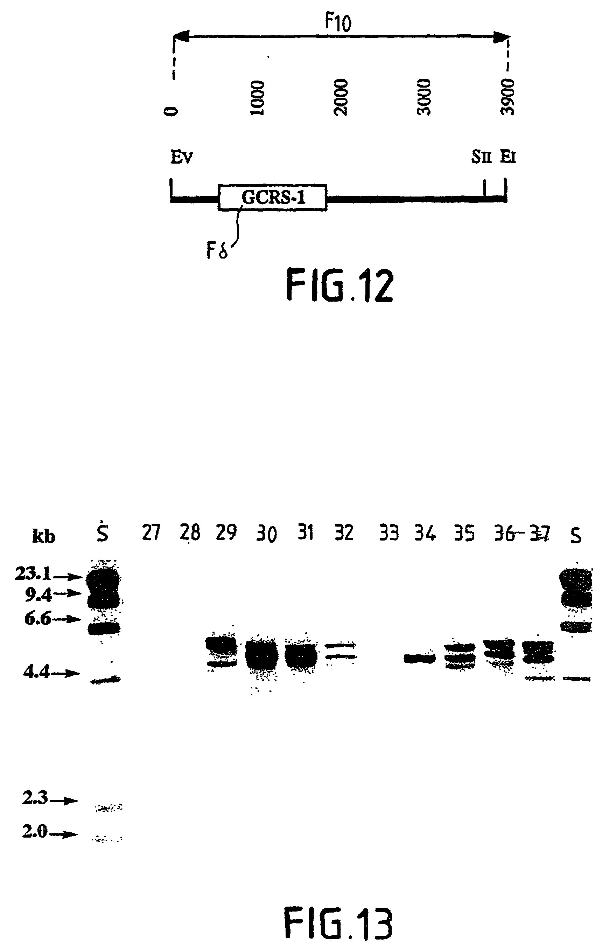

La **Figure 12** donne la carte de restriction du fragment F10 comprenant la séquence-source itérative Fδ (GCRS-1).
Abréviations : EV = *Eco*RV, SII = *Sac*II, EI = *Eco*RI.

### 7.2. Test de détection/identification

La **Figure 13** montre les résultats d'hybridation, selon la méthode de Southern, de l'ADN de souches des espèces *Geotrichum candidum et Geotrichum fermentans* (digérés par *Msp*I) avec la sonde A₂O₂₇ spécifique de *Geotrichum candidum*. Les fragments de restriction par *Msp*I de l'ADN des souches étudiées ont été séparés par électrophorèse en gel d'agarose à 0,8 % puis transférés sur membrane en nylon. La membrane a été sondée avec environ 100 ng de la sonde A₂O₂₇. Cette sonde n'hybride qu'avec l'ADN des souches de *Geotrichum candidum* et révèle un polymorphisme de la taille des fragments de restriction.
La sonde A₂O₂₇ a été développée selon le modèle de la sonde F = F1, Fα, Fβ, Fγ1, Fγ2. La sonde A₂O₂₇ est constituée d'une partie F₁₀ dont Fδ de l'IGR₁ d'une souche de *Geotrichum candidum*. La sonde A₂O₂₇ hybride uniquement avec l'ADN des souches de *Geotrichum candidum* (**Fig. 13**). Elle n'hybride pas avec l'ADN des souches de *Geotrichum fermentans*, bien que ces deux espèces soient proches. Cet exemple montre bien la spécificité de cette sonde selon l'invention. De plus, cette sonde révèle également un polymorphisme de la taille des fragments de restriction selon les souches de *Geotrichum candidum*. Ceci permet de les différencier entre-elles et donc de les typer.
Cet exemple illustre la validité du modèle présenté de sonde d'identification-typage pour les levures d'intérêt médical et/ou d'intérêt industriel.
Les souches référencées sur la **Figure 13**, N° 27 à 37 correspondant à :
S = Standard
G. *fermentans* CBS 50.57
G. *fermentans* CBS 25.29
*G. candidum* F
G. *candidum* A
G. candidum B
*G. candidum* LMGC3
G. *fermentans*
G. *candidum* 149.26
G. *candidum* LMGC8
G. *candidum* LMGC7
G. *candidum* CBS 557.83
Standard

## Revendications

1. Sonde de détection spécifique et/ou infraspécifique de levures, du type de celle choisie parmi les outils génétiques suivants : au moins une partie :
* d'au moins un fragment F d'acide nucléique ADN, construit et/ou isolé à partir d'au moins une séquence-source d'ADN ribosomal (ADNr) :
localisée dans la région d'espacement intergénique (IGR) non transcrite (NTS) et comprise entre l'ADNr codant pour la sous-unité 25 S d'ARNr, et l'ADNr codant pour la sous-unité 18 S d'ARNr,
* et/ou d'au moins un analogue Fa de ce fragment, résultant de la dégénérescence du code génétique ;
* et/ou d'au moins un fragment Fc d'ADNc complémentaire du fragment F,
au moins une partie des produits de transcription primaire du fragment F, Fa et/ou Fc.
**caractérisé en ce que** la séquence-source d'ADNr pour F et/ou Fa et/ou Fc est sélectionnée de telle sorte
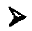 qu'elle soit itérative
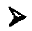 qu'elle comprenne au moins 2, de préférence de 2 à 12 sous-séquences répétées,
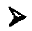 et que le degré d'homologie entre ces sous-séquences soit supérieur ou égal à 50 %.

2. Sonde selon la revendication 1, **caractérisée en ce que** le degré d'homologie est supérieur ou égal à 55%.

3. Sonde selon la revendication 1, **caractérisée en ce que** le degré d'homologie est supérieur ou égal à 60%.

4. Sonde selon l'une quelconque des revendications 1 à 3, **caractérisée en ce que** la séquence-source de F est localisée dans la région d'espacement intergénique 2 (IGR₂) non transcrite (NTS) et comprise entre l'ADNr codant pour la sous-unité 5S d'ARNr et l'ADNr codant pour la sous-unité 18 S d'ARNr.

5. Sonde selon la revendication 4, **caractérisée en ce que** la séquence-source itérative de F comprend au moins trois sous-séquences répétées comportant chacune au moins 80 bases nucléotidiques.

6. Sonde selon la revendication 5, **caractérisée en ce que** lesdites séquences répétées comportent chacune au moins 120 bases nucléotidiques.

7. Sonde selon la revendication 6, **caractérisée en ce que** lesdites séquences répétées comportent chacune entre 150 et 300 bases nucléotidiques.

8. Sonde selon l'une quelconque des revendications 1 à 7, **caractérisée en ce que** la séquence-source itérative de F comprend au moins une sous-séquence de longueur tronquée par rapport aux autres sous-séquences.

9. Sonde selon la revendication 8, **caractérisée en ce que** la sous-séquence tronquée est terminale.

10. Sonde selon l'une quelconque des revendications 1 à 4, **caractérisée en ce que** la séquence-source itérative de F comprend au moins trois sous-séquences répétées comportant chacune au moins 4 bases nucléotidiques ; chaque sous-séquence comprenant au moins un motif commun à toutes les sous-séquences et ayant une longueur inférieure ou égale à celle de la sous-séquence à laquelle il appartient.

11. Sonde selon la revendication 10, **caractérisée en ce que** lesdites séquences répétées comportent chacune au moins 6 bases nucléotidiques.

12. Sonde selon la revendication 11, **caractérisée en ce que** lesdites séquences répétées comportent chacune entre 7 et 25 bases nucléotidiques.

13. Sonde selon la revendication 1, **caractérisée en ce que** la séquence-source de F :
• est localisée dans la région d'espacement intergénique 1 (IGR₁) non transcrite (NTS) et comprise entre l'ADNr codant pour la sous-unité 5S d'ARNr et l'ADNr codant pour la sous-unité 25 S d'ARNr
• et comprend au moins trois sous-séquences répétées comportant chacune au moins 4 bases nucléotidiques, chaque sous-séquence comprenant au moins un motif commun à toutes les sous-séquences et ayant une longueur inférieure ou égale à celle de la sous-séquence à laquelle il appartient.

14. Sonde selon la revendication 13, **caractérisée en ce que** lesdites sous-séquences répétées comportent chacune au moins 5 bases nucléotidiques.

15. Sonde selon la revendication 14, **caractérisée en ce que** lesdites sous-séquences répétées comportent chacune entre 5 et 50 bases nucléotidiques.

16. Sonde selon la revendication 15, **caractérisée en ce que** lesdites sous-séquences répétées comportent chacune entre 5 et 40 bases nucléotidiques.

17. Sonde selon la revendication 13, **caractérisée en ce que** la séquence source de F correspond à au moins deux séquences ayant respectivement de 5 à 20 et de 9 à 25 bases nucléotidiques.

18. Séquence d'ADN **caractérisée en ce qu'**elle est choisie dans le groupe des séquences annexées suivantes : SEQ ID NO : 1 : ; SEQ ID NO : 2 : ; SEQ ID NO : 3 : ; SEQ ID NO : 4 : et SEQ ID NO : 5 :.

19. Sonde selon au moins l'une des revendications 1 à 15, **caractérisée en ce que** la séquence-source de F correspond à :
SEQ ID NO : 2 : ;
SEQ ID NO : 3 : ;
SEQ ID NO : 4 : et
SEQ ID NO : 5 :.

20. Sonde selon la revendication 1, **caractérisée :**
- **en ce qu'**elle comprend au moins une partie d'au moins l'une des séquences sources de F telles que définies aux revendications 5 à 7, 10 à 17 et 19,
- **en ce qu'**elle est appliquée :
▷ à la détection/identification spécifique et infraspécifique de levures,
▷ ou comme marqueur épidémiologique,
▷ ou encore comme traceur de souche,
▷ ou bien enfin dans le cadre d'une stratégie thérapeutique visant des affections à levures.

21. Sonde selon la revendication 20, **caractérisée en ce qu'**elle comprend au moins une partie de l'une des séquences sources de F telles que définies aux revendications 5 à 7.

22. Procédé de détection et d'identification de levures **caractérisé en ce qu'**il consiste à mettre en oeuvre au moins une sonde selon l'une quelconque des revendications 1 à 17 et 19 à 21 et comprend les étapes consistant à
- extraire l'ADN total génomique des souches à étudier,
- soumettre éventuellement cet ADN total à une digestion enzymatique à l'aide d'au moins une enzyme de restriction,
- dénaturer l'ADN total éventuellement digéré,
- mettre en présence l'ADN total ainsi dénaturé, avec la sonde dotée d'au moins un marqueur, de façon à réaliser l'hybridation,
- éliminer l'ADN et la sonde non hybridée, et
- révéler l'hybridation à l'aide du marqueur.

23. Amorce oligonucléotidique, utilisable notamment pour la réaction de polymérisation en chaîne (PCR), **caractérisée en ce qu'**elle est constituée par la sonde selon l'une quelconque des revendications 1 à 17 et 19.

24. Application de la sonde selon l'une quelconque des revendications 1 à 17 et 19 à 21, pour la détection, l'identification inter-espèces et/ou la différenciation infraspécifique des levures notamment des espèces *Candida krusei* ou *Geotrichum candidum*.

## Patentansprüche

1. Nachweissonde, die für Hefen spezifisch und/oder intraspezifisch ist, eines solchen Typs, der ausgewählt ist aus den folgenden genetischen Hilfsmitteln: wenigstens einem Teil von:
- wenigstens einem Fragment F einer Nukleinsäure DNA, das konstruiert wurde und/oder ausgehend von wenigstens einer Ursprungssequenz ribosomaler DNA (rDNA) isoliert wurde:
das in dem nicht transkribierten (NTS) intergenen Bereichsabschnitt (IGR) lokalisiert ist und zwischen der rDNA, die für die Untereinheit 25 S der rRNA codiert, und der rDNA, die für die Untereinheit 18 S der rRNA codiert, enthalten ist,
- und/oder wenigstens einem Analogen Fa dieses Fragments, das aus der Degeneriertheit des genetischen Codes resultiert;
- und/oder wenigstens einem Fragment Fc einer cDNA, das zu dem Fragment F komplementär ist,
wenigstens einem Teil der primären Transkriptionsprodukte der Fragmente F, Fa und/oder Fc,
**dadurch gekennzeichnet, dass** die Ursprungssequenz der rDNA für F und/oder Fa und/oder Fc ausgewählt ist aus solchen,
- die iterativ sind
- die wenigstens zwei, vorzugsweise 2 bis 12 wiederholte Teilsequenzen enthalten,
- und wobei der Homologiegrad zwischen diesen Teilsequenzen größer oder gleich 50 % ist.

2. Sonde nach Anspruch 1, **dadurch gekennzeichnet, dass** der Homologiegrad größer oder gleich 55 % ist.

3. Sonde nach Anspruch 1, **dadurch gekennzeichnet, dass** der Homologiegrad größer oder gleich 60 % ist.

4. Sonde nach irgendeinem der Ansprüche 1 bis 3, **dadurch gekennzeichnet, dass** die Ursprungssequenz von F in dem nicht transkribierten (NTS) intergenen Bereichsabschnitt 2 (IGR₂) lokalisiert ist und zwischen der rDNA, die für die Untereinheit 5 S der rRNA codiert, und der rDNA, die für die Untereinheit 18 S der rRNA codiert, enthalten ist.

5. Sonde nach Anspruch 4, **dadurch gekennzeichnet, dass** die iterative Ursprungssequenz von F wenigstens drei wiederholte Teilsequenzen umfasst, die jeweils wenigstens 80 Nukleotidbasen aufweisen.

6. Sonde nach Anspruch 5, **dadurch gekennzeichnet, dass** die wiederholten Sequenzen jeweils wenigstens 120 Nukleotidbasen aufweisen.

7. Sonde nach Anspruch 6, **dadurch gekennzeichnet, dass** die wiederholten Sequenzen jeweils zwischen 150 und 300 Nukleotidbasen aufweisen.

8. Sonde nach irgendeinem der Ansprüche 1 bis 7, **dadurch gekennzeichnet, dass** die iterative Ursprungssequenz von F wenigstens eine Teilsequenz umfasst, die im Vergleich zu anderen Teilsequenzen eine verkürzte Länge aufweist.

9. Sonde nach Anspruch 8, **dadurch gekennzeichnet, dass** die verkürzte Teilsequenz terminal ist.

10. Sonde nach irgendeinem der Ansprüche 1 bis 4, **dadurch gekennzeichnet, dass** die iterative Ursprungssequenz von F wenigstens drei wiederholte Teilsequenzen umfasst, die jeweils wenigstens 4 Nukleotidbasen aufweisen; wobei jede Teilsequenz wenigstens ein Motiv umfasst, das allen Teilsequenzen gemeinsam ist und eine Länge aufweist, die kürzer ist oder gleich jener der Teilsequenz, welcher dieses angehört.

11. Sonde nach Anspruch 10, **dadurch gekennzeichnet, dass** die wiederholten Sequenzen jeweils wenigstens 6 Nukleotidbasen aufweisen.

12. Sonde nach Anspruch 11, **dadurch gekennzeichnet, dass** die wiederholten Sequenzen jeweils zwischen 7 und 25 Nukleotidbasen aufweisen.

13. Sonde nach Anspruch 1, **dadurch gekennzeichnet, dass** die Ursprungssequenz von F:
- in dem nicht transkribierten (NTS) intergenen Bereichsabschnitt 1 (IGR₁) lokalisiert ist und zwischen der rDNA, die für die Untereinheit 5 S der rRNA codiert, und der rDNA, die für die Untereinheit 25 S der rRNA codiert, enthalten ist
- und wenigstens drei wiederholte Teilsequenzen umfasst, die jeweils wenigstens 4 Nukleotidbasen aufweisen, wobei jede Teilsequenz wenigstens ein Motiv umfasst, das allen Teilsequenzen gemeinsam ist und eine Länge aufweist, die kürzer ist oder gleich jener der Teilsequenz, welcher dieses angehört.

14. Sonde nach Anspruch 13, **dadurch gekennzeichnet, dass** die wiederholten Teilsequenzen jeweils wenigstens 5 Nukleotidbasen aufweisen.

15. Sonde nach Anspruch 14, **dadurch gekennzeichnet, dass** die wiederholten Teilsequenzen jeweils zwischen 5 und 50 Nukleotidbasen aufweisen.

16. Sonde nach Anspruch 15, **dadurch gekennzeichnet, dass** die wiederholten Teilsequenzen jeweils zwischen 5 und 40 Nukleotidbasen aufweisen.

17. Sonde nach Anspruch 13, **dadurch gekennzeichnet, dass** die Ursprungssequenz von F wenigstens zwei Sequenzen entspricht, welche. 5 bis 20 bzw. 9 bis 25 Nukleotidbasen aufweisen.

18. DNA-Sequenz, **dadurch gekennzeichnet, dass** diese ausgewählt ist aus der Gruppe der folgenden angefügten Sequenzen: SEQ ID NO: 1; SEQ ID NO: 2; SEQ ID NO: 3; SEQ ID NO: 4 und SEQ ID NO: 5.

19. Sonde nach wenigstens einem der Ansprüche 1 bis 15, **dadurch gekennzeichnet, dass** die Ursprungssequenz von F entspricht:
SEQ ID NO: 2;
SEQ ID NO: 3;
SEQ ID NO: 4; und
SEQ ID NO: 5.

20. Sonde nach Anspruch 1, **dadurch gekennzeichnet, dass**:
- diese wenigstens einen Teil wenigstens einer der Ursprungssequenzen von F, wie diese in den Ansprüchen 5 bis 7, 10 bis 17 und 19 definiert sind, umfasst,
- diese angewendet wird für:
- den spezifischen und intraspezifischen Nachweis/Identifizierung von Hefen,
- oder als epidemiologischer Marker,
- oder auch als Tracer für einen Stamm,
- oder aber im Rahmen einer therapeutischen Behandlung im Hinblick auf Erkrankungen durch Hefen.

21. Sonde nach Anspruch 20, **dadurch gekennzeichnet, dass** diese wenigstens einen Teil einer der Ursprungssequenzen von F, wie diese in den Ansprüchen 5 bis 7 definiert sind, umfasst.

22. Verfahren des Nachweisens und der Identifizierung von Hefen, **dadurch gekennzeichnet, dass** dieses aus dem Anwenden wenigstens einer Sonde nach irgendeinem der Ansprüche 1 bis 17 und 19 bis 21 besteht und die folgenden Schritte umfasst:
- Extrahieren der gesamten genomischen DNA der zu untersuchenden Stämme,
- gegebenenfalls Unterwerfen dieser gesamten DNA unter einen enzymatischen Verdau mit Hilfe wenigstens eines Restriktionsenzyms,
- Denaturieren der gegebenenfalls verdauten gesamten DNA,
- Zusammenbringen der so denaturierten gesamten DNA mit der Sonde, die mit wenigstens einem Marker ausgestattet ist, um eine Hybridisierung durchzuführen,
- Entfernen der DNA und der nicht hybridisierten Sonde und
- Aufzeigen der Hybridisierung mit Hilfe des Markers.

23. Oligonukleotidprimer, der insbesondere für die Polymerasekettenreaktion (PCR) verwendbar ist, **dadurch gekennzeichnet, dass** dieser aus der Sonde nach irgendeinem der Ansprüche 1 bis 17 und 19 besteht.

24. Verwendung der Sonde nach irgendeinem der Ansprüche 1 bis 17 und 19 bis 21 für den Nachweis, die Interspezies-Identifizierung und/oder die intraspezifische Differenzierung der Hefen insbesondere der Spezies *Candida krusei* oder *Geotrichum candidum.*

## Claims

1. Probe for the specific and/or infraspecific detection of yeasts, of the type selected from the following genetic tools: at least part:
* of at least one DNA nucleic acid fragment F constructed and/or isolated from at least one source sequence of ribosomal DNA (rDNA):
located in the non-transcribed (NTS) intergenic spacer region (IGR) between the rDNA coding for the subunit 25S of rRNA and the rDNA coding for the subunit 18S of rRNA,
* and/or of at least one analog Fa of this fragment resulting from the degeneracy of the genetic code,
* and/or of at least one cDNA fragment Fc complementary to the fragment F,
at least part of the primary transcription products of the fragment F, Fa and/or Fc,
**characterized in that** the source sequence of rDNA for F and/or Fa and/or Fc is selected so that
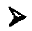 it is iterative,
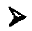 it comprises at least 2 and preferably from 2 to 12 repeat subsequences,
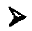 and the degree of homology between these subsequences is greater than or equal to 50%.

2. Probe according to claim 1, **characterized in that** the degree of homology is greater than or equal to 55%.

3. Probe according to claim 1, **characterized in that** the degree of homology is greater than or equal to 60%.

4. Probe according to any one of claims 1 to 3, **characterized in that** the source sequence of F is located in the non-transcribed (NTS) intergenic spacer region 2 (IGR₂) between the rDNA coding for the subunit 5S of rRNA and the rDNA coding for the subunit 18S of rRNA.

5. Probe according to claim 4, **characterized in that** the iterative source sequence of F comprises at least three repeat subsequences each containing at least 80 nucleotide bases.

6. Probe according to claim 5, **characterized in that** said repeat sequences each contain at least 120 nucleotide bases.

7. Probe according to claim 6, **characterized in that** said repeat sequences each contain between 150 and 300 nucleotide bases.

8. Probe according to any one of claims 1 to 7, **characterized in that** the iterative source sequence of F comprises at least one subsequence of truncated length compared with the other subsequences.

9. Probe according to claim 8, **characterized in that** the truncated subsequence is terminal.

10. Probe according to any one of claims 1 to 4, **characterized in that** the iterative source sequence of F comprises at least three repeat subsequences each containing at least 4 nucleotide bases, each subsequence comprising at least one unit common to all the subsequences and having a length less than or equal to that of the subsequence to which it belongs.

11. Probe according to claim 10, **characterized in that** said repeat sequences each contain at least 6 nucleotide bases.

12. Probe according to claim 11, **characterized in that** said repeat sequences each contain between 7 and 25 nucleotide bases.

13. Probe according to claim 1, **characterized in that** the source sequence of F:
• is located in the non-transcribed (NTS) intergenic spacer region 1 (IGR₁) between the rDNA coding for the subunit 5S of rRNA and the rDNA coding for the subunit 25S of rRNA,
• and comprises at least three repeat subsequences each containing at least 4 nucleotide bases, each subsequence comprising at least one unit common to all the subsequences and having a length less than or equal to that of the subsequence to which it belongs.

14. Probe according to claim 13, **characterized in that** said repeat subsequences each contain at least 5 nucleotide bases.

15. Probe according to claim 14, **characterized in that** said repeat subsequences each contain between 5 and 50 nucleotide bases.

16. Probe according to claim 15, **characterized in that** said repeat subsequences each contain between 5 and 40 nucleotide bases.

17. Probe according to claim 13, **characterized in that** the source sequence of F corresponds to at least two sequences having from 5 to 20 and from 9 to 25 nucleotide bases, respectively.

18. DNA sequence, **characterized in that** it is selected from the following group of attached sequences: SEQ ID NO : 1 :, SEQ ID NO : 2 :, SEQ ID NO : 3 :, SEQ ID NO : 4 : and SEQ ID NO : 5 :.

19. Probe according to at least one of claims 1 to 15, **characterized in that** the source sequence of F corresponds to:
SEQ ID NO : 2 :,
SEQ ID NO : 3 :,
SEQ ID NO : 4 : and
SEQ ID NO : 5 :.

20. Probe according to claim 1, **characterized in that**:
- it comprises at least part of at least one of the source sequences of F as defined in claims 5 to 7, 10 to 17 and 19,
- and it is applied:
▷ to the specific and infraspecific detection/identification of yeasts,
▷ or as an epidemiological marker,
▷ or as a strain tracer,
▷ or, finally, in the context of a therapeutic strategy aimed at diseases caused by yeasts.

21. Probe according to claim 20, **characterized in that** it comprises at least part of one of the source sequences of F as defined in claims 5 to 7.

22. Method of detecting and identifying yeasts, **characterized in that** it consists in using at least one probe according to any one of claims 1 to 17 and 19 to 21 and comprises the steps which consist in
- extracting the total genomic DNA from the strains to be studied,
- optionally subjecting this total DNA is to enzymatic digestion with at least one restriction enzyme,
- denaturing the total DNA which may have been digested,
- bringing the denatured total DNA into contact with the probe, carrying at least one marker, so as to effect the hybridization,
- removing the DNA and the non-hybridized probe, and
- disclosing the hybridization with the aid of the marker.

23. Oligonucleotide primer usable especially for the polymerase chain reaction (PCR), **characterized in that** it consists of the probe according to any one of claims 1 to 17 and 19.

24. Application of the probe according to any one of claims 1 to 17 and 19 to 21 to the detection, interspecies identification and/or infraspecific differentiation of yeasts, especially the species *Candida krusei* or *Geotrichum candidum.*
